(19) Europäisches Patentamt
European Patent Office
Office européen des brevets

(11) EP 0 717 040 A1

(12) EUROPEAN PATENT APPLICATION

(43) Date of publication:
19.06.1996 Bulletin 1996/25

(51) Int. Cl.[6]: C07D 279/06, A61K 31/54, C07D 281/02

(21) Application number: 95112468.4

(22) Date of filing: 08.08.1995

(84) Designated Contracting States:
AT BE CH DE DK ES FR GB GR IE IT LI LU MC NL PT SE

(30) Priority: 14.12.1994 JP 333321/94
07.04.1995 JP 108032/95
17.05.1995 JP 144134/95

(71) Applicant: Japan Tobacco Inc.
Minato-Ku Tokyo 105 (JP)

(72) Inventors:
- Yata, Shinji,
c/o Central Pharmaceutical Res.
Takatsuki-shi, Osaka, 569 (JP)
- Ozeki, Hidekazu,
c/o Central Pharmaceutical Res.
Takatsuki-shi, Osaka, 569 (JP)
- Wakitani, Korekiyo,
c/o Central Pharmaceutical Res
Takatsuki-shi, Osaka, 569 (JP)

(74) Representative: Reinhard - Skuhra - Weise & Partner
Postfach 44 01 51
80750 München (DE)

# (54) Thiazine or thiazepine derivatives which inhibit NOS

(57) A thiazine- or a thiazepine-derivative represented by the following general formula (1), or a pharmaceutically acceptable salt thereof.

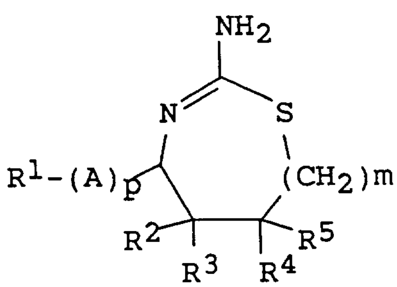

(I)

wherein $R^1$ is a hydrogen atom, a substituted or unsubstituted alkyl group, a cycloalkyl group, a substituted or unsubstituted alkenyl group, a substituted or unsubstituted alkynyl group, a substituted or unsubstituted aryl group, a substituted or unsubstituted heteroaryl group, or a substituted or unsubstituted aralkyl group, $R^2$, $R^3$, $R^4$, and $R^5$, which may be the same or different, respectively are a hydrogen atom, a substituted or unsubstituted alkyl group, A is an oxygen atom, a sulfur atom, or an NH group, m is 0 or 1, and p is 0 or 1, This compound has a nitric oxide synthase inhibition activity.

EP 0 717 040 A1

## Description

The present invention relates to novel thiazine derivatives or thiazepine derivatives which have an excellent nitric oxide synthase inhibition activity. The invention further relates to a pharmaceutical composition which comprising a thiazine- or thiazepine-derivative and a pharmaceutically acceptable carrier, the derivative including the novel compound. The pharmaceutical composition is useful as an anti-inflammatory agent, a shock-treatment agent and an ischemic encephalopathy treatment agent depending upon the nitric oxide synthase inhibition activity of the aforementioned derivative.

Nitric oxide (NO) synthesized by a nitric oxide synthase (NOS) has been proven to participate in a wide variety of in-vivo biological reaction processes, as will be described below.

In 1980, Furchgott et al. reported that an endothelium-derived relaxing factor (EDRF) has a strong vasodilating activity and a platelet aggregation-inhibitory activity. In 1987, Palmer et al. reported that the entity of EDRF is nitric oxide (NO).

Thereafter, NO was found to be produced not only in vascular endothelial cells, but also in all tissue cells throughout a body, for example, cerebellum, platelets, peripheral nerve, macrophages, polynuclear leucocytes, hepatic cells, Kupffer's cells, renal mesangial cells, pulmonary parenchymal cells, adrenal vascular smooth muscle, fibrobrasts, and the like. It was further elucidated that the NO function include not only a vascular smooth muscle relaxation activity, which is function primary elucidated, but also a neurotransmitter activity and a cytotoxic activity against bacteria and tumor cells.

On the other hand, it is reported that when NO is excessively produced and released in a living body, it causes disorders in various cells and tissues due to the vascular relaxation activity thereof as well as high reactivity ascribed to its chemical lability (J. Natl. Cancer Inst., 84, 27 (1992), Lancet, 338, 1555(1991)).

NO is produced by an enzymatic reaction of an NO synthase (NOS) using L-arginine as a substrate. Hitherto, NO synthases (NOSs) have been purified from several types of cells, and their cDNA sequences have been clarified. NOSs can be roughly classified into two types, a constitutive NOS called cNOS and an inducible NOS called iNOS on the basis of differences in the expression patterns and their cofactor demands. The cNOSs are further divided into a neuronal NOS (nNOS) distributed in nervous tissues and an endothelium NOS (eNOS) distributed in vascular endothelium cells.

The cNOS distributed in cells, such as vascular endothelium and cerebellum is $Ca^{2+}$/calmodulin-dependent and produces NO in a short period of time by responding to agonist's stimulation. The NO thus formed mediates transmission of information via various physiological reactions.

In contrast, iNOS distributed in macrophages, gliocytes, leucocytes, hepatic cells, vascular smooth muscle cells, and the like, is produced by endotoxin (LPS), various types of cytokines (IFN$\gamma$, TNF, ILs etc.,), and the like.

iNOS, once expressed, produces NO over a long period of time. However, unlike cNOS, iNOS is considered to be $Ca^{2+}$/calmodulin-independent and to contribute to cytotoxicity. It is conceivable that NO excessively produced by iNOS is deeply related to the onset pathology of diseases such as endotoxic/hemorrhagic shock.

Very recently, it was confirmed that an L-arginine analogue serving as an NOS inhibitor has an effect of improving cerebral edema and cerebral infarction in an animal model of encephaloischemia (Neurosci. Lett., 147, 159-162 (1992)).

Accordingly, compounds efficiently inhibiting these NOSs (NOS inhibitors), if obtained, will be advantageous in treating disorders, which are caused presumably by excessively produced and released NO. Examples of the disorders are: various types of cardiopathy and encephalopathy ascribed to infarction and ischemia, in particular, encephalopathy caused by ischemia; shock such as endotoxic/hemorrhagic shock and cardiac shock; and inflammatory disorders such as acute inflammations, chronic disorders attributable to antoimmune diseases e.g., rheumatism. Particularly, these compounds are effective to the treatment for ischemic encephalopathy, in other words, various disorders associated with cerebral ischemia and post-ischemia reperfusion injury.

Known NOS inhibitors are, for example, various L-arginine analogues (Jpn. Pat. Appln. KOKAI Publication Nos. 4-270255 and 5-286916, Jpn. Pat. Appln. KOHYO Publication No. 5-500659, and WO93/13055), and imidazoline derivatives (Jpn. Pat. Appln. KOKAI Publication No. 6-211805).

Quite lately, it was found that a straight-chain isothiourea derivative, and 5-membered heterocyclic compounds such as 2-aminothiazole compound and 2-aminothiazoline compound exhibit the NOS inhibition activity (WO94/12165, J. Biol. Chem., 269(43), 26669-26676 (1994)).

On the other hand, as 2-amino-1,3-thiazine derivative which is 6-membered heterocyclic compound, the following compounds are known in various technical fields. However, none of these compounds are suggested to be the NOS inhibitor.

For example, the following compound is described as an intermediate compound to produce 5,6-dihydro-2H-[1,2,4]-thiodiazole[3,2-b]thiazine-2-one in J. Org. Chem., 38(8), 1575-1578(1973):

(a)

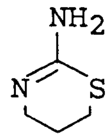

A synthesis method for the following compound is described in Khim Geterotsikl. Soedin., page 1572-1575, No. 11, 1987:

(b)

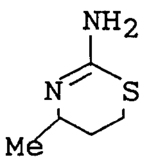

The following compound is described to have an analgesic effect, in CA. 62, 13152g and the US patent publication No. 3169090:

(C)

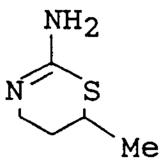

The following compound is disclosed as a developing agent for developing an electrostatic image in Jpn. Pat. Appln. KOKAI Publication No. 60-57852:

(d)

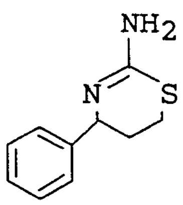

The following compound is disclosed as a developing agent in Jpn. Pat. Appln. KOKAI Publication No. 59-197053:

(e) 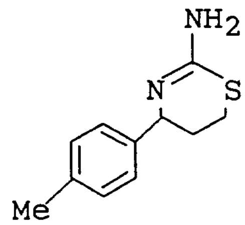

The following compound is disclosed as an intermediate of an indoleamine-N-methyltransferase inhibitor in Jpn. Pat. Appln. KOKAI Publication No. 52-153975.

(f) 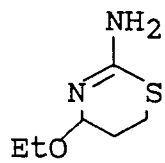

Whereas, the aforementioned prior art does not teach or suggest the NOS inhibitor.

As described above, the NOS inhibitor comprising a conventionally known L-arginine analogue, 2-aminothiazole compound or 2-aminothiazoline compound has been proven not enough to be used practically due to its low activity or side effect. More specifically, since the L-arginine analog has a structural similarity to essential amino acid L-arginine, it may adversely affect on normal metabolic systems other than the NO synthesis in a living body. On the other hand, the 2-aminothiazole compound scarcely possesses a desired NOS inhibition activity, and the 2-aminothiazoline compound does not possess a sufficient activity, as is apparent from the results of comparative examples described later. Hence, a novel NOS inhibitor has been desired which has a higher activity and a structure entirely different from that of L-arginine.

The present invention has been made in view of the aforementioned circumstances. A first object of the present invention is to provide a novel compound having a higher NOS inhibition activity and a structure entirely different from that of L-arginine.

A second object of the present invention is to provide a pharmaceutical composition which comprises the compound having aforementioned NOS inhibition activity as an active ingredient, the active ingredient compound including the novel compound.

A third object of the present invention is to provide a method of treating a variety of diseases, by using the aforementioned pharmaceutical composition.

The present inventors have intensively studied and investigated with the view toward attaining the aforementioned objects. As a result, they unexpectedly found that a compound having a 6-membered heterocyclic thiazine skeleton or a 7-membered heterocyclic thiazepine skeleton, possesses an extremely excellent NOS inhibition activity, thereby accomplishing the present invention.

To describe more specifically, the first object of the present invention can be attained by a 2-amino-5,6-dihydro-4H-1,3-thiazine derivative, or a 2-amino-4,5,6,7-tetrahydro-1,3-thiazepine derivative represented by the following formula (I), or a pharmaceutically acceptable salt thereof:

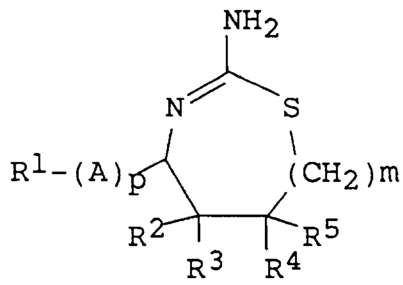

(I)

wherein

- $R^1$ is a hydrogen atom, a substituted or unsubstituted alkyl group, a cycloalkyl group, a substituted or unsubstituted alkenyl group, a substituted or unsubstituted alkynyl group, a substituted or unsubstituted aryl group, a substituted or unsubstituted heteroaryl group, or a substituted or unsubstituted aralkyl group;
- $R^2$, $R^3$, $R^4$, and $R^5$, which may be the same or different and each is a hydrogen atom, a substituted or unsubstituted alkyl group, or $R^1$ together with $R^2$ or $R^3$ may represent

$$-(CH_2)_n-$$

where n is an integer from 1 to 6;
- A is an oxygen atom, a sulfur atom, or an NH group;
- m is 0 or 1; and
- p is 0 or 1,

with the proviso that the following compounds (a) to (f), is excluded from the scope of the Formula (1).

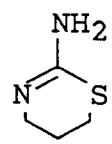 , 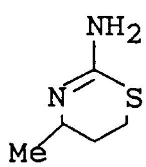 , 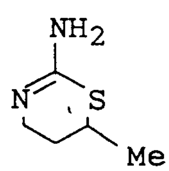 , 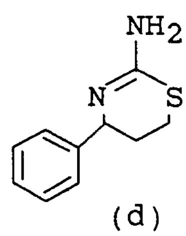 ,

(a) (b) (c) (d)

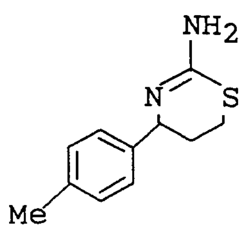 , 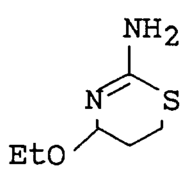

(e) (f)

The second object of the present invention can be attained by using a pharmaceutical composition containing a compound represented by the aforementioned formula (I) or a pharmaceutically acceptable salt thereof in an amount sufficient to inhibit an NOS activity, and a pharmaceutically acceptable carrier, wherein $R^1$, $R^2$, $R^3$, $R^4$, $R^5$ A, m and p in the formula (I) respectively have the same meanings as defined above with the provide that the case where m and p are simultaneously 0; $R^1$, $R^2$, $R^3$, $R^4$ are simultaneously a hydrogen atom; and $R^5$ is a methyl group is excluded. It should

be noted that in the pharmaceutical composition, the compound (c) is only excluded from the scope of the active ingredients, but the compounds (a), (b), (d) - (f) are not.

The third object of the present invention is attained by a method of treating a subject suffering from an abnormal symptom caused by an excessive level of in-vivo nitric oxide synthesis, which comprises administering a compound represented by the afore-mentioned general formula (I) or a pharmaceutically acceptable salt thereof in an amount sufficient to inhibit the NOS activity, wherein $R^1$, $R^2$, $R^3$, $R^4$, $R^5$, A, m and p in the formula (I) have the same meanings as defined above respectively. In this case, the aforementioned compounds (a) to (f) are all included in the compound represented by general formula (I).

⟨Novel Compound⟩:

(1) As described above, the novel compound of the present invention is a 2-amino-5,6-dihydro-4H-1,3-thiazine derivative, or a 2-amino-4,5,6,7-tetrahydro-1,3-thiazepine derivative represented by the following formula (I), or a pharmaceutically acceptable salt thereof.

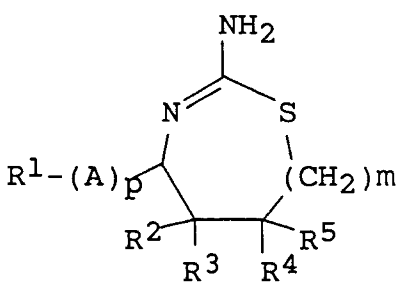

(I)

wherein

- $R^1$ is a hydrogen atom, a substituted or unsubstituted alkyl group, a cycloalkyl group, a substituted or unsubstituted alkenyl group, a substituted or unsubstituted alkynyl group, a substituted or unsubstituted aryl group, a substituted or unsubstituted heteroaryl group, or a substituted or unsubstituted aralkyl group;
- $R^2$, $R^3$, $R^4$, and $R^5$, which may be the same or different, respectively are a hydrogen atom, a substituted or unsubstituted alkyl group, or $R^1$ together with $R^2$ or $R^3$ may represent

$$-(CH_2)_n-$$

where n is an integer from 1 to 6;
- A is an oxygen atom, a sulfur atom, or an NH group;
- m is 0 or 1; and
- p is 0 or 1,
with the proviso that the following compounds (a) to (f) are excluded from the scope of the Formula (I).

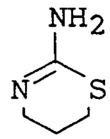,
(a)

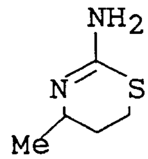,
(b)

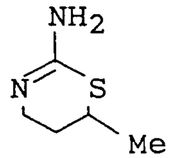,
(c)

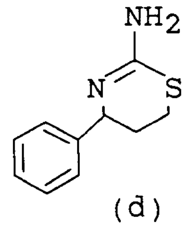,
(d)

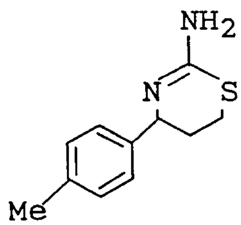,
(e)

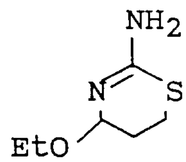
(f)

These compounds (a) to (f) are excluded since they are already known as compounds per se.

The compounds of the present invention mentioned above may have stereoisomers, optical isomers and tautomers. All of these isomers are included in the present invention.

The term "alkyl group" used herein means a straight-chain or branched-chain alkyl group having 1 to 6 carbon atoms. Examples of, the alkyl groups include methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, isohexyl, 2-hexyl, and the like. Preferable alkyl group is a lower alkyl group having 1 to 4 carbon atoms, for example, methyl, ethyl, n-propyl, isopropyl, n-butyl, isobutyl, sec-butyl, tert-butyl, or the like.

The particularly preferable group as $R^1$ is a lower alkyl group which may be branched, such as methyl, ethyl, n-propyl, isopropyl, n-butyl, or isobutyl. $R^2$ to $R^5$ are preferably lower alkyl groups, more preferably, methyl or ethyl.

The phrase "substituted or unsubstituted alkyl group" used herein means that the alkyl group defined above may be substituted with one or more substituents described below. The substituents include a hydroxyl group, halogen atom, amino group, nitro group, primary or secondary alkylamino group, cyano group, cycloalkyl group, alkoxyl group, carboxyl group, alkoxycarbonyl group, and the like. The substituent in $R^1$ is preferably a hydroxyl group, halogen atom, amino group, cycloalkyl group, alkoxyl group, cyano group, carboxyl group, alkoxycarbonyl group, or the like, more preferably, a hydroxyl, amino, cycloalkyl, alkoxyl, carboxyl, alkoxycarbonyl group, or the like. Preferable substituent in $R^2$ to $R^5$ is a hydroxyl group, alkoxyl group, halogen atom, amino group, cycloalkyl group, carboxyl group, or alkoxycarbonyl group. It should be noted that the substitution site of the substituent in the substituted alkyl group and the number thereof are not particularly restricted.

The term "halogen atom" used herein means a fluorine, chlorine, bromine or iodine atom, preferably a fluorine, chlorine or bromine atom. The particularly preferable halogen atom as the substituent in $R^1$ is a fluorine or chlorine atom. The particularly preferable halogen atom as the substituent in $R^2$ to $R^5$ is a chlorine or bromine atom.

The phrase "primary or secondary alkylamino group" used herein means an amino group which is mono- or disubstituted with the alkyl group defined above. Examples of the primary or secondary alkylamino groups include methylamino, ethylamino, n-propylamino, isopropylamino, n-butylamino, isobutylamino, sec-butylamino, tert-butylamino, n-pentylamino, isopentylamino, tert-pentylamino, neopentylamino, n-hexylamino, isohexylamino, dimethylamino, diethylamino, di-n-propylamino, diisopropylamino, di-n-butylamino, diisobutylamino, methylethylamino, methyl-n-butylamino and the like. Preferably, the primary or secondary alkylamino groups are methylamino, ethylamino, n-propylamino, isopropylamino, n-butylamino, isobutylamino, dimethylamino, diethylamino, di-n-propylamino, diisopropylamino, di-n-butylamino, diisobutylamino or the like. If circumstances require, the primary or secondary alkylamino group may form a heterocyclic ring, such as an azetidino, pyrrolidino, piperidino, morpholino group, or the like. Of all the aforementioned alkylamino groups, the particularly preferable substituent in $R^1$ is methylamino, ethylamino, dimethylamino, diethylamino, di-n-propylamino, diisopropylamino, or the like. The particularly preferable substituent in $R^2$ to $R^5$ is a methylamino, ethylamino, dimethylamino, diethylamino, or the like.

The term "cycloalkyl group" used herein means a cyclic alkyl group having 3 to 8 carbon atoms. Examples of the cycloalkyl groups include cyclopropyl, cyclobutyl, cyclopentyl, cyclohexyl, cycloheptyl, cyclooctyl, and the like. A preferable cycloalkyl group is one having 3 to 6 carbon atoms such as cyclopropyl, cyclobutyl, cyclopentyl, cyclohexyl or the like. Of all the aforementioned cycloalkyl groups, the particularly preferable substituent in $R^1$ is cyclopropyl, cyclobutyl, cyclopentyl, cyclohexyl or the like. The particularly preferable substituent in $R^2$ to $R^5$ is a cyclobutyl, cyclopentyl or cyclohexyl group or the like.

The term "alkoxyl group" used herein means a straight-chain or branched-chain alkoxyl group having 1 to 6 carbon atoms. Examples of the alkoxyl groups include methoxy, ethoxy, n-propoxy, isopropoxy, n-butoxy, isobutoxy, sec-butoxy, tert-butoxy, n-pentoxy, isopentoxy, tert-pentoxy, neopentoxy, n-hexoxy, isohexoxy, and the like. Preferable alkoxyl group is a lower alkoxyl group having 1 to 4 carbon atoms, such as methoxy, ethoxy, n-propoxy, isopropoxy, n-butoxy, isobutoxy, sec-butoxy, tert-butoxy, or the like. The particularly preferable alkoxyl group as the substituent in $R^1$ is methoxy, ethoxy, n-propoxy, isopropoxy, or the like. The particularly preferable alkoxyl groups as the substituent in $R^2$ to $R^5$ are methoxy, ethoxy, or the like.

The term "alkoxycarbonyl group" used herein means an alkoxycarbonyl group whose alkyl moiety is that of a straight-chain or branched-chain having 1 to 6 carbon atoms. Examples of the alkoxycarbonyl group include methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentoxycarbonyl, isopentoxycarbonyl, tert-pentoxycarbonyl, neopentoxycarbonyl, n-hexoxycarbonyl, isohexoxycarbonyl, and the like. A preferable alkoxycarbonyl group is one which comprises an alkyl moiety having 1 to 4 carbon atoms, such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, or the like. The particularly preferable substituent in $R^1$ is a methoxycarbonyl, ethoxycarbonyl, n-propoxycarbonyl, isopropoxycarbonyl, or the like. The particularly preferable substituents in $R^2$ to $R^5$ are a methoxycarbonyl, ethoxycarbonyl group, or the like.

The term "alkenyl group" used herein means a straight-chain or branched-chain alkenyl group having 2 to 6 carbon atoms. Examples of the alkenyl groups include ethenyl, 1-propenyl, 2-propenyl, 1-butenyl, 2-butenyl, 3-butenyl, 2-methyl-1-propenyl, 1-pentenyl, 2-pentenyl, 3-pentenyl, 2-methyl-1-butenyl, 3-methyl-1-butenyl, 1-hexenyl, 2-hexenyl, 3-hexenyl, 4-hexenyl, 5-hexenyl, 2-methyl-1-pentenyl, 3-methyl-1-pentenyl, 4-methyl-1-pentenyl, 2,3-dimethyl-1-butenyl, 3,3-dimethyl-1-butenyl, and the like. The alkenyl group is preferably one having 1 to 4 carbon atoms, such as ethenyl, 1-propenyl, 2-propenyl, 1-butenyl, 2-butenyl, 3-butenyl, 2-methyl-1-propenyl, or the like; more preferably, ethenyl, 1-propenyl, 2-propenyl, 1-butenyl, 2-butenyl, 3-butenyl, or the like.

The term "substituted alkenyl group" used herein means the alkenyl group having one or more substituents defined with regard to the aforementioned "substituted or unsubstituted alkyl group". The substituent is preferably a halogen atom, cyano group, alkoxyl group, alkoxycarbonyl group, or the like, more preferably, an alkoxyl group, alkoxycarbonyl group, or the like. It should be noted that the substitution site of a substituent in the alkenyl group and the number thereof are not particularly restricted.

The term "alkynyl group" used herein is a straight-chain or branched-chain alkynyl group having 2 to 6 carbon atoms. Examples of the alkynyl groups include ethynyl, 1-propynyl, 2-propynyl, 1-butynyl, 2-butynyl, 3-butynyl, 1-methyl-2-propynyl, 1-pentynyl, 2-pentynyl, 3-pentynyl, 1-methyl-2-butynyl, 1-methyl-3-butynyl, 2-methyl-3-butynyl, 1-hexynyl, 2-hexynyl, 3-hexynyl, 4-hexynyl, 5-hexynyl, 3-methyl-1-pentynyl, 4-methyl-1-pentynyl, 1-methyl-2-pentynyl, 1-methyl-3-pentynyl, 1-methyl-4-pentynyl, 1,1-dimethyl-3-butynyl, 3,3-dimethyl-1-butynyl, and the like. The ethynyl group is preferably one having 1 to 4 carbon atoms, such as ethynyl, 1-propynyl, 2-propynyl, 1-butynyl, 2-butynyl, 3-butynyl, 1-methyl-2-propynyl, or the like, more preferably, ethynyl, 1-propynyl, 2-propynyl, 1-butynyl, 2-butynyl, 3-butynyl, or the like.

The term "substituted alkynyl group" used herein means the alkynyl group having a substituent defined with regard to the aforementioned "substituted or unsubstituted alkyl group". The substituent is preferably a halogen atom, cyano group, alkoxyl group, alkoxycarbonyl group, or the like, more preferably, an alkoxyl group, or alkoxycarbonyl group. It should be noted that the substitution site of the substituent and the number thereof are not particularly restricted.

The term "substituted or unsubstituted aryl group" used herein means an aryl group which may have one or more substituents on its ring such as phenyl, naphthyl, biphenyl, or the like. Examples of the substituents include an alkyl group, hydroxyl group, halogen atom, amino group, nitro group, primary or secondary alkylamino group, cyano group, cycloalkyl group, haloalkyl group, alkoxyl group, carboxyl group, alkoxycarbonyl group, and the like. The substituent is preferably an alkyl group, hydroxyl group, halogen atom, amino group, nitro group, alkoxyl group, or the like; more preferably, an alkyl group, halogen atom, alkoxyl group, or the like. It should be noted that the substitution site of the substituent and the number thereof are not particularly restricted. However, mono- to tri-substitution is preferable, and mono-substitution is more preferable.

The term "haloalkyl group" used herein means an alkyl group substituted with one or more halogen atoms. In the halogen atom, the halogen atom defined with regard to aforementioned "halogen atom" can be included. The alkyl group in the haloalkyl includes that defined with respect to the aforementioned "lower alkyl group". It should

be noted that the substitution site of a halogen atom and the number thereof are not particularly restricted. Examples of the haloalkyl groups include fluoromethyl, trifluoromethyl, chloromethyl, dichloromethyl, trichloromethyl, bromomethyl, 2-fluoromethyl, 2,2,2-trifluoroethyl, 2-chloroethyl, 2,2,2-trichloroethyl, 2-bromoethyl, 3-fluoropropyl, 3-chloropropyl, 3-bromopropyl, and the like.

The term "substituted or unsubstituted heteroaryl group" used herein means a heteroaryl group which may have one or more substituents on its ring. Examples of the heteroaryl groups include pyridyl, pyrimidyl, pyrazinyl, franyl, thienyl, pyrrolyl, imidazolyl, oxazolyl, isooxazolyl, thiazolyl, isothiazolyl, indolyl, isoindolyl, quinolyl, isoquinolyl, phthalazinyl, naphthyridinyl, quinoxalinyl, quinazolinyl, cinnolinyl, and the like. As the substituents, the substituent defined with respect to "substituted or unsubstituted aryl group" can be mentioned. The substituent is preferably an alkyl group, hydroxyl group, halogen atom, amino group, nitro group, alkoxyl group, carboxyl group, alkoxycarbonyl group; more preferably, a hydroxyl group, halogen atom, primary or secondary alkyl amino group, or alkoxyl group. It should be noted that the substitution site of the substituents and the number thereof are not particularly restricted. However, the substitution is preferably mono- to tri-substitution, more preferably mono-substitution.

The term "substituted or unsubstituted aralkyl group" used herein means an aryl-alkyl group having an aryl moiety such as phenyl, naphthyl, or biphenyl, and an alkyl group moiety having 1 to 6 carbon atoms, the aryl moiety may having one or more substituents on a ring. Examples of the substituents include an alkyl group, hydroxyl group, halogen atom, alkoxyl group, haloalkyl group, nitro group, amino group, cyano group, and the like. The number of the substituents are preferably 1 to 3. More preferable, aralkyl group is that comprising a phenyl group moiety which may be mono- to tri-substituted with a halogen atom, alkyl group, alkoxyl group or the like, and comprising an alkyl group moiety having 1 to 4 carbon atoms. Examples of the aralkyl groups include benzyl, phenethyl, phenylpropyl, phenylbutyl, phenylhexyl, and the like. It should be noted that the substitution site on the aryl ring is not particularly restricted. Examples of preferable aralkyl groups include benzyl, halogen-substituted benzyl, alkyl-substituted benzyl, alkoxyl-substituted benzyl, phenethyl, halogen-substituted phenethyl, alkyl-substituted phenethyl, alkoxyl-substituted phenethyl, phenylpropyl, halogen-substituted phenylproply, alkyl-substituted phenylpropyl, alkoxyl-substituted phenylpropyl, and the like. The particularly preferable example is benzyl, phenethyl, or the like.

The term "pharmaceutically acceptable salt" used herein means a non-toxic salt formed between a thiazine- or a thiazepine-derivative represented by the aforementioned formula (1) and an appropriate acid. As the appropriate acid, any acid can be used as long as it forms the non-toxic salt. Examples of the acids include inorganic acids such as hydrochloric acid, sulfuric acid, phosphoric acid, hydrobromic acid, and nitric acid; and organic acids such as acetic acid, propionic acid, oxalic acid, malonic acid, citric acid, lactic acid, malic acid, succinic acid, tartaric acid, fumaric acid, maleic acid, glycolic acid, methanesulfonic acid, p-toluenesulfonic acid, gluconic acid, ascorbic acid, aspartic acid, and glutamic acid. If the circumstances require, the salt may be a hydrous form or a hydrate.

Hereinbelow, we will mention preferable embodiments of the novel compound of the present invention described in detail above.

(2) The thiazine derivative, thiazepine derivative or pharmaceutically acceptable salt thereof according to the aforementioned (1), in which the thiazine- or the thiazepine-derivative is selected from the group consisting of the following compounds.

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;
- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;

- 2-amino-4-benzyloxy-5,6-dihydro-4H-1,3-thiazine;
- 2-amino-4-cyclopropylmethoxy-5,6-dihydro-4H-1,3-thiazine;
- 4-allyloxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;
- 2-amino-5,6-dihydro-5-methyl-4H-1,3-thiazine; and
- 2-amino-5,6-dihydro-6,6-dimethyl-4H-1,3-thiazine.

(3) The thiazine derivative, thiazepine derivative, or pharmaceutically acceptable salt thereof according to the aforementioned (2), in which the thiazine- or thiazepine-derivative is selected from the group consisting of the following compounds.

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

(4) The thiazine derivative, thiazepine derivative or pharmaceutically acceptable salt thereof according to the aforementioned (3), in which the thiazine- or thiazepine-derivative is selected from the group consisting of the following compounds.

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

(5) The thiazine derivative, thiazepine derivative, or pharmaceutically acceptable salt thereof according to the aforementioned (4), in which the thiazine- or thiazepine-derivative is selected from the group consisting of the following compounds.

- (R)-2-amino-5,6-dihydro-4-propyl-4H-1,3-thiazine; and
- (S)-2-amino-5,6-dihydro-4-methoxymethyl-4H-1,3-thiazine.

(6) The thiazine derivative or pharmaceutically acceptable salt thereof according to the above mentioned (5), in which the thiazine derivative is (R)-2-amino-5,6-dihydro-4- propyl-4H-1,3-thiazine.
(7) The thiazine derivative, or pharmaceutically acceptable salt thereof according to the above (5), in which the thiazine derivative is (S)-2-amino-5,6-dihydro-4- methoxymethyl-4H-1,3-thiazine.

⟨pharmaceutical composition⟩:

(8) The pharmaceutical composition of the present invention contains a sufficient amount of a thiazine derivative or a thiazepine derivative represented by the following general formula (I) or a pharmaceutically acceptable salt thereof to inhibit the NOS activity, and a pharmaceutically acceptable carrier.

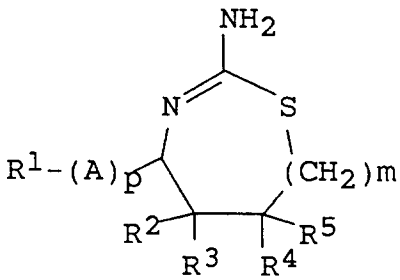

(I)

wherein $R^1$, $R^2$, $R^3$, $R^4$, $R^5$ A, m and p respectively have the same meanings as defined above with the proviso that the formula (I) does not include the case where m and p are simultaneously 0; $R^1$, $R^2$, $R^3$, $R^4$ are simultaneously a hydrogen atom; and $R^5$ is a methyl group. In the case of the pharmaceutical composition, excluded from the formula (I) is only the following compound (c):

(C)

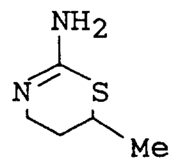

On the other hand, the aforementioned compounds (a), (b) (d) - (f) are not excluded, because only the compound (c) is known as an active ingredient of the pharmaceutical composition, and the pharmaceutical compositions using compounds (a) (b), (d) - (f) as an active ingredient have not yet been known.

"Pharmaceutically acceptable carrier" is known in the art and can be appropriately selected to use depending upon respective dosage forms of the pharmaceutical composition.

When the pharmaceutical composition of the present invention is made into a solid composition for oral administration, possible dosage forms are tablets, pills, powders, granules, and the like. In such a solid composition, at least one of active ingredients is mixed with at least one of inert diluents, dispersants or adsorbents, such as lactose, mannitol, glucose, hydroxypropylcellulose, microcrystalline cellulose, starch, polyvinylhydrine, magnesium aluminate metasilicate, or anhydrous silicic acid powder. Alternatively, to the composition, one or more additives other than a diluent may be added according to a conventional method.

In the case where tablets or pills are prepared, if necessary, they may be coated with a film which is soluble in a stomach or an intestine, such as white sugar, gelatin, hydroxypropylcellulose or hydroxymethylcellulose phthalate. They may be coated with at least two film layers. Alternatively, the composition may be encapsulated with a material such as gelatin or ethylcellulose.

In the case where liquid compositions are prepared for oral administration, possible administration forms are a pharmaceutically acceptable emulsion, solution, suspension, syrup, elixir, or the like. Examples of usable diluents include purified water, ethanol, vegetable oil, emulsifier, or the like. To this composition, auxiliary agents other than a diluent, such as a moistening agent, suspending agent, sweetener, flavor, perfume, or antiseptic may be added.

In the case where parenterally injectable preparations are prepared, an aqueous or non-aqueous sterile solution, solubilizer, suspending agent or emulsifying agent is used. Examples of the aqueous solutions, solubilizers, and suspending agents include distilled water for injection, a saline solution, cyclodextrin and a derivative thereof, organic amines such as triethanolamine, diethanolamine, monoethanolamine, and triethylamine, and an inorganic alkaline solution, and the like.

In the case where liquid preparations soluble in water are prepared, for example, propyleneglycol, polyethyleneglycol, or a vegetable oil such as olive oil, alcohols such as ethanol, and the like, may be used. As the solubilizer, for example, a surfactant (which forms a mixed micell) such as polyoxyethylene hydrogenated castor oil, and fatty acid ester of sucrose; lecithin or hydrogenated lecithin (which forms a riposome), or the like is used. On the other hand, emulsions can be made of non-aqueous solbilizer such as a vegetable oil, lechthin, polyoxyethylene hydrogenated caster oil or polyoxyethylenepolyoxypropyleneglycol and the like.

As other preparations for non-oral administration, there may be a liquid for external use, a liniment such as an ointment, a suppository, or a pessary containing at least one active substance and formulated in accordance with a known method.

The pharmaceutical composition of the present invention has pharmacological effect such as antiinflammatory activity, angiopathy-suppressing activity, and vasopressor activity ascribed to the NO synthase inhibition activity. On the basis of the pharmacological activities, the pharmaceutical composition of the present invention is advantageous in treating various diseases presumably caused by NO excessively produced and released. Examples of the diseases include encephalopathy and cardiopathy ascribed to infarction and ischemia, in particular, encephalopathy caused by ischemia; shock such as endotoxic, hemorrhagic and cardiac shock; and inflammatory disorders such as acute inflammations and chronic disorders attributable to antoimmune diseases, e.g., rheumatism. Particularly, these compounds are advantageous to the treatment for ischemic encephalopathy, in other words, various disorders accompanying cerebral ischemia and post-ischemia reperfusion injury.

When the pharmaceutical composition of the present invention is used as an antiinflammatory agent, a shock treatment agent and an agent for treating ischemic encephalopathy including a cerebral ischemia and a ischemic reperfusion injury, in usual, it is administered to a patient orally or non-orally, as well as in systemic or local manner.

The dosages is varied depending on age, body weight and symptom of the patient, treatment effect, administration method, treatment time, and the like. Usually, the daily dosage of the antiinflammatory agent is in the range from 10 mg to 1 g per an adult. The daily dosage of the shock treatment agent is in the range from 0.01 mg to 100 mg, and that of the ischemic encephalopathy treatment agent is in the range 0.01 to 10 mg. These agents are orally or non-orally administered one to several times a day.

Hereinbelow, preferable embodiments of the pharmaceutical composition of the present invention will be described.

(9) The pharmaceutical composition according to the aforementioned (8), wherein in formula (1), p is 0; and $R^1$ represents a hydrogen atom, a substituted or unsubstituted alkyl group, a substituted or unsubstituted aryl group, a substituted or unsubstituted aralkyl group, or $R^1$ together with $R^2$ forms

$$-(CH_2)_n-$$

(n is the same as defined above).

(10) The pharmaceutical composition according to the aforementioned (8), in which in formula (1), p is 1; each of $R^2$, $R^3$ and $R^5$ is a hydrogen atom; and $R^4$ is a hydrogen atom or a substituted or unsubstituted alkyl group.

(11) The pharmaceutical composition according to the aforementioned (8), in which the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;
- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;
- 2-amino-4-ethoxy-5,6-dihydro-4H-1,3-thiazine;
- 2-amino-4-benzyloxy-5,6-dihydro-4H-1,3-thiazine;
- 2-amino-4-cyclopropylmethoxy-5,6-dihydro-4H-1,3-thiazine;
- 4-allyoxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;

- 2-amino-5,6-dihydro-5-methyl-4H-1,3-thiazine; and
- 2-amino-5,6-dihydro-6,6-dimethyl-4H-1,3-thiazine.

(12) The pharmaceutical composition according to the aforementioned (11), in which the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

(13) The pharmaceutical composition according to the aforementioned (12), in which the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

(14) The pharmaceutical composition according to the aforementioned (13), in which the thiazine- or thiazepine-derivative is selected from the group consisting of the following compounds:

- (R)-2-amino-5,6-dihydro-4-propyl-4H-1,3-thiazine; and
- (S)-2-amino-5,6-dihydro-4-methoxymethyl-4H-1,3-thiazine.

(15) The pharmaceutical composition according to the aforementioned (14), in which thiazine- or a thiazepine-derivative is (R)-2-amino-5,6-dihydro-4-propyl-4H-1,3-thiazine.
(16) The pharmaceutical composition according to the aforementioned (14), in which thiazine- or a thiazepine-derivative is (S)-2-amino-5,6-dihydro-4-methoxymethyl-4H-1,3-thiazine.

⟨Therapeutic method⟩

(17) The therapeutic method of the present invention is a method of treating a subject suffering from an abnormal symptom ascribed to an excessive level of in-vivo nitric oxide synthesis, which comprises administering a compound represented by the following formula (I) or a pharmaceutically acceptable salt thereof in an amount sufficient to inhibit the NOS activity of the subject, thereby down-regulating the nitric oxide level of the subject:

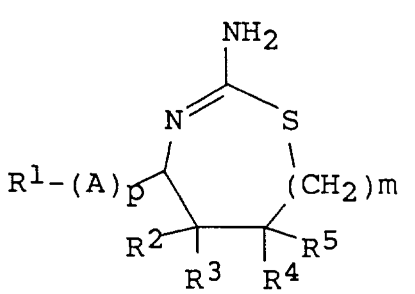

(I)

where $R^1$, $R^2$, $R^3$, $R^4$, $R^5$, A, m and p respectively have the same meanings as defined above.

The therapeutic method of the present invention is concerned with the use of a compound represented by the formula (I). More specifically, this therapeutic method is based on the finding that the compound represented by the formula (I) can inhibit the NOS activity, thereby down regulating an excessive in-vivo NO level. Such the activity has

not been known to any one of the compounds represented by the formula (I). Accordingly, the compounds (a) to (f), which are excluded from the compounds represented by the formula (I) with respect to the novel compounds of the present invention, are not excluded here.

The therapeutic method of the present invention, since it is based on the NOS inhibition activity of the compound represented by the formula (I), is advantageous in treating various disorders presumably caused by excessively produced and released NO, such as various types of cardiopathy and encephalopathy ascribed to infarction and ischemia, in particular, encephalopathy caused by ischemia; shock such as endotoxic, hemorrhagic and cardiac shock; and inflammatory disorders such as acute inflammations, and chronic disorders attributable to antoimmune diseases, e.g., rheumatism. Particularly, these compounds are advantageous to the treatment for ischemic encephalopathy, in other words, various disorders accompanying cerebral ischemia and post-ischemia reperfusion injury.

When the method of the present invention is practiced, the compounds of the formula (I) is administered to a subject in a form of pharmaceutical composition mixed with a pharmaceutically acceptable carrier. The dosage form and administration route are the same as described with respect to a pharmaceutical composition of the present invention.

Based on the fact that therapies for a variety of disorders can be effective as described above using the compound of formula (I), the present invention further provides the use of the compound represented by formula (I) in preparing a medicament for treating these disorders.

More specifically, the present invention provides the use of a compound represented by the following formula (I) or a pharmaceutically acceptable salt thereof in preparing a medicament for treating a subject suffering from an abnormal symptom ascribed to an excessive level of in-vivo nitric oxide synthesis:

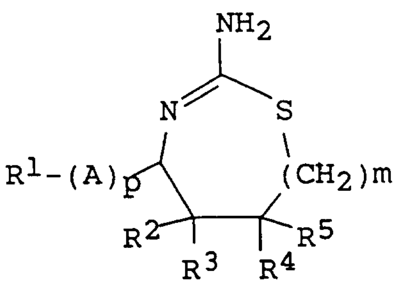

(I)

where $R^1$, $R^2$, $R^3$, $R^4$, $R^5$ A, m, p respectively have the same meanings as defined above.

Hereinbelow, preferred embodiments of the therapeutic method and use of the present invention will be described.

(18) The therapeutic method or use according to the aforementioned (17), wherein in the formula (I), p is 0; $R^1$ is a hydrogen atom, a substituted or unsubstituted alkyl group, a substituted or unsubstituted aryl group, a substituted or unsubstituted aralkyl group, or $R^1$ together with $R^2$ forms

$$-(CH_2)_n-$$

(n is the same as defined above).

(19) The therapeutic method or use according to the aforementioned (17), wherein in the general formula (I), p is 1; each of $R^2$, $R^3$ and $R^5$, is a hydrogen atom; and $R^4$ is a hydrogen atom or a substituted or unsubstituted alkyl group.

(20) The therapeutic method or use according to the aforementioned (17), wherein the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;

- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;
- 2-amino-4-ethoxy-5,6-dihydro-4H-1,3-thiazine;
- 2-amino-4-benzyloxy-5,6-dihydro-4H-1,3-thiazine;
- 2-amino-4-cyclopropylmethoxy-5,6-dihydro-4H-1,3-thiazine;
- 4-allyloxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;
- 2-amino-5,6-dihydro-5-methyl-4H-1,3-thiazine; and
- 2-amino-5,6-dihydro-6,6-dimethyl-4H-1,3-thiazine.

(21) The therapeutic method or use according to the aforementioned (20), wherein the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

(22) The therapeutic method or use according to the aforementioned (21), in which the thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (R)-2-amino-5,6-dihydro-4-isobutyl-4H-1,3-thiazine; and
- (S)-2-amino-5,6-dihydro-4-methoxymethyl-4H-1,3-thiazine.

(23) The therapeutic method or use according to the aforementioned (22), wherein the thiazine- or thiazepine-derivative is selected from the group consisting of the following compounds:

- (R)-2-amino-5,6-dihydro-4-propyl-4H-1,3-thiazine; and
- (S)-2-amino-5,6-dihydro-4-methoxymethyl-4H-1,3-thiazine.

(24) The therapeutic method or use according to the aforementioned (23), wherein the thiazine- or thiazepine-derivative is (R)-2-amino-5,6-dihydro-4-propyl-4H-1,3-thiazine.
(25) The therapeutic method or use according to the aforementioned (23), wherein the thiazine- or the thiazepine-derivative is (S)-2-amino-5,6-dihydro-4-methoxymethyl-4H-1,3-thiazine.
(26) The therapeutic method or use according to the aforementioned (17), wherein the abnormal symptom ascribed to an excessive level of in-vivo NO synthesis is inflammation.
(27) The therapeutic method or use according to any one of the aforementioned (18) to (25), wherein the abnormal symptom ascribed to an excessive level of in-vivo NO synthesis is inflammation.
(28) The therapeutic method or use according to the aforementioned (17), wherein the abnormal symptom ascribed to an excessive level of in-vivo NO synthesis is a shock symptom.

(29) The therapeutic method or use according to any one of the aforementioned (18) to (25), wherein the abnormal symptom ascribed to an excessive level of in-vivo NO synthesis is a shock symptom.

(30) The therapeutic method or use according to the aforementioned (17), wherein the abnormal symptom ascribed to an excessive level of in-vivo NO synthesis is ischemic encephalopathy.

(31) The therapeutic method or use according to any one of the aforementioned (18) to (25), wherein the abnormal symptom ascribed to an excessive level of in-vivo NO synthesis is ischemic encephalopathy.

⟨Synthesis of the Compound (I)⟩:

Hereinbelow, the method of preparing the compound according to the present invention will be described; however, it should not be limited to the methods described below.

In the following explanation, "amino-protecting group" may be any protecting group as long as it is usually used as a protecting group for an amino group. Examples of the amino-protecting groups include an alkoxycarbonyl group such as a methoxycarbonyl, ethoxycarbonyl, propoxycarbonyl, isopropoxycarbonyl, n-butoxycarbonyl, isobutoxycarbonyl, sec-butoxycarbonyl, tert-butoxycarbonyl, tert-amyoxycarbonyl, 2,2,2-trichloroethoxycarbonyl, benzyloxycarbonyl, or 9-fluorenylmethoxycarbonyl; an acyl group such as formyl, acetyl, propionyl, butyroyl, valeryl, pivaloyl, chloroacetyl, trichloroacetyl, trifluoroacetyl or benzoyl; an alkyl group or an aralkyl group such as methyl, tert-butyl, or benzyl group. The amino-protecting group is preferably a methoxycarbonyl, ethoxycarbonyl, tert-butoxycarbonyl, benzyloxycarbonyl, formyl, acetyl, pivaloyl, benzoyl, or tert-butyl group; more preferably, a tert-butoxycarbonyl, benzoyl, or tert-butyl group.

As the "carboxyl-protecting group", any protecting group is available as long as it is usually used as a protecting group for a carboxyl group. Examples of the carboxyl-protecting groups include an alkyl group such as methyl, ethyl, n-propyl, isopropyl, n-butyl, isobutyl or tert-butyl; an halogen-substituted alkyl group such as 2,2,2-trichloroethyl or 2,2,2-trifluoroethyl; an aralkyl group such as benzyl, p-methoxybenzyl, 3,4-dimethoxybenzyl, o-nitrobenzyl, p-nitrobenzyl, p-bromobenzyl, diphenylmethyl, or trityl; a silyl group such as trimethylsilyl, triethylsilyl, isopropyldimethylsilyl, tert-butylmethylsilyl, di-tert-butylmethylsilyl, phenyldimethylsilyl or tert-butyldiphenylsilyl; or allyloxycarbonyl. The carboxyl-protecting group is preferably an alkyl group such as a methyl, ethyl, or tert-butyl and an aralkyl group such as benzyl, or p-methoxybenzyl, more preferably a tert-butyl.

The "leaving group" is an atom or an atomic group leaving from a carbon atom of the molecue to which the leaving group is bonded, together with an electron pair. Any leaving group is available as long as it is used usually in a synthetic chemical reaction. Examples of the leaving groups include a halogen such as fluorine, chlorine, bromine, or iodine; and a sufonyloxy group such as a methansulfonyloxy, trifluoromethansulfonyloxy, benzenesulfonyloxy, p-toluenesulfonyloxy, p-bromobenzenesulfonyloxy group. The leaving group is preferably chlorine, bromine, iodine, as well as a methansulfonyloxy group, trifluoromethanesulfonyloxy group, or p-toluenesulfonyloxy group, more preferably, chlorine, bromine, methansulfonyloxy group, or a p-toluenesulfonyloxy group.

<u>Preparation Method A</u>

Hereinbelow, we will explain a method for preparing a compound represented by formula (I) in which <u>m</u> is 0, <u>p</u> is 0, and $R^3$ and $R^5$ respectively represent hydrogen atoms; or in which <u>m</u> is 1, <u>p</u> is 0; and $R^3$ and $R^5$ are represent hydrogen atom.

Chemical formula 10

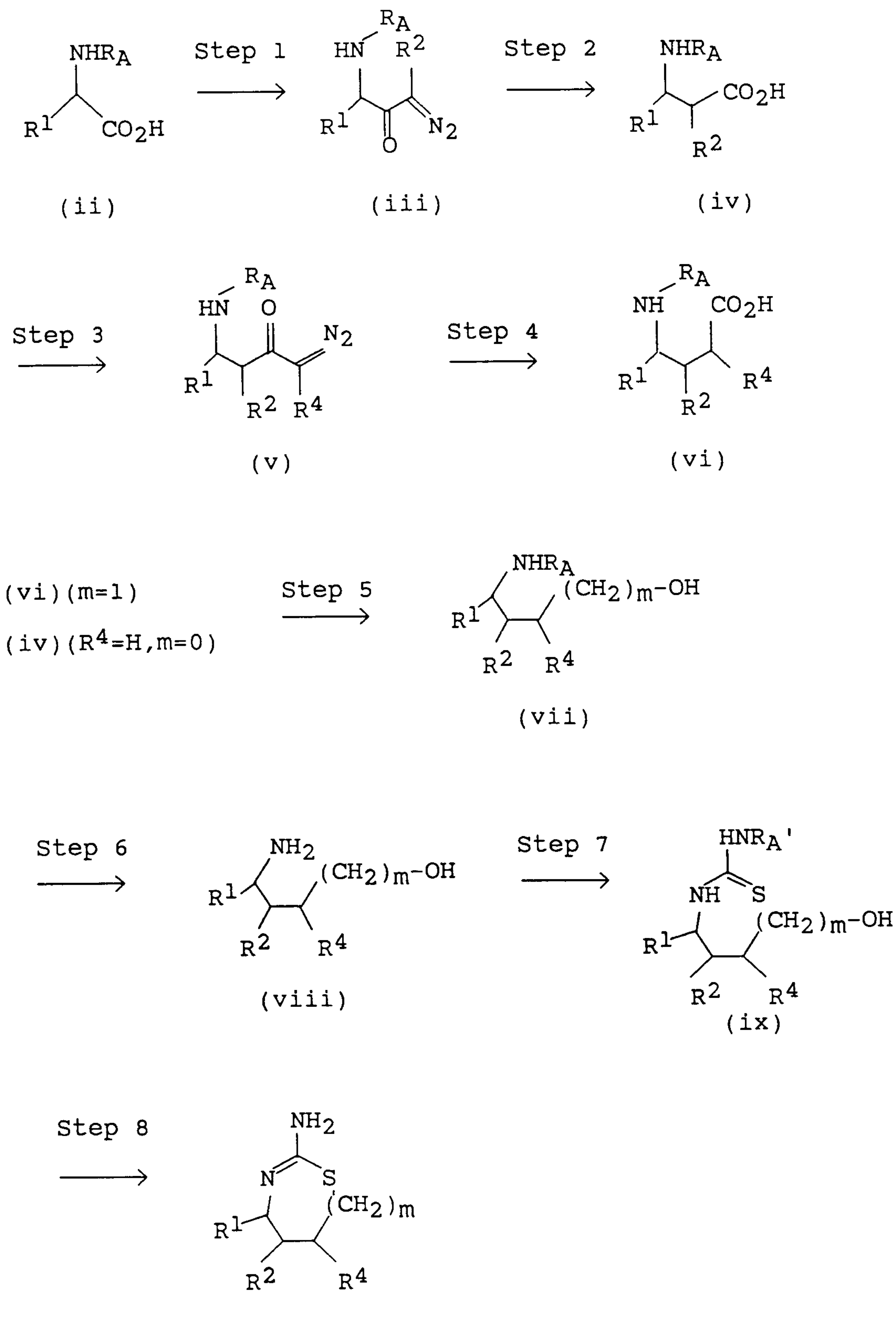


(ii) (iii) (iv)

(v) (vi)

(vi)(m=1)

(iv)($R^4$=H,m=0)

(vii)

(viii) (ix)

(I-1)

〈Step 1〉

A conventionally known amino acid derivative or a compound readily prepared from known compounds which are represented by formula (ii) (in which $R^1$ represent the same meaning as defined above and $R_A$ represent an amino-protecting group) is reacted with an acid halide such as pivaloyl chloride or tosyl chloride, or with an alkyl halocarbonate such as ethyl chlorocarbonate or isobutyl chlorocarbonate, in an inert solvent not preventing the reaction such as diethylether, tetrahydrofuran, or 1,4-dioxane, at the temperature from -20 to 40°C, in the presence of a base such as pyridine, triethylamine, N,N-diisopropylethylamine or N-methylmorphorine, thereby affording a mixed acid anhydride.

Alternatively, the compound of the formula (ii) may be reacted with a halogenating agent such as thionyl chloride or oxalyl chloride at the temperature from -20 to 40°C, to give an acid halide. In the case of preparing the acid halide, the reaction may be carried out with no solvent or in the presence of a base which is used in the case of preparing the aforementioned mixed acid anhydride.

Subsequently, these products obtained above are reacted with diazoalkane such as diazomethane or a derivative thereof obtained in accordance with a known method, in the aforementioned inert solvent, at the temperature from -20°C to room temperature, preferably under ice-cooling. As a result, a compound represented by formula (iii) (in which $R^1$, $R^2$, and $R_A$ respectively represent the same meanings as defined above) is obtained.

〈Step 2〉

A compound represented by formula (iii) is subjected to the reaction at the temperature from room temperature to reflux temperature, in water or a mixed solvent between water and an organic solvent such as tetrahydrofuran, 1,4-dioxane, acetone, acetonitrile and N, N-dimethylformamide, in the presence of a catalytic amount of an alkaline solution of silver salt such as a triethylamine solution of silver benzoate, an aqueous ammonia solution of silver oxide. As a result, a compound represented by formula (iv) ($R^1$, $R^2$ and $R_A$ respectively represent the same meanings as defined above) is obtained.

Thereafter, in preparation of a desired compound represent by formula (iv) in which $R^4$ is an hydrogen atom and <u>m</u> is 0, the reaction is allowed to proceed to Step 5 without experiencing Steps 3 and 4.

〈Step 3〉

A compound represented by formula (iv) is reacted with diazomethane or the like in the same manner as that described in Step 1, thereby affording a compound represented by formula (v) ($R^1$, $R^2$, $R^4$ and $R_A$ respectively represent the same meanings as defined above).

〈Step 4〉

A compound represented by formula (v) is subjected to the same reaction as described in Step 2, thereby affording a compound represented by formula (vi) ($R^1$, $R^2$, $R^4$ and $R_A$ respectively represent the same meanings as defined above).

〈Step 5〉

A compound represented by formula (iv) or (vi) is reduced with a reducing agent such as a lithium aluminum hydride or a borane-tetrahydrofuran complex, in an inert solvent such as tetrahydrofuran at the temperature from -20°C to reflux temperature. As a result, a compound represented by formula (vii) ($R^1$, $R^2$, $R^4$, $R_A$ and <u>m</u> respectively have the same meanings as defined above) can be obtained. Alternatively, the compound of formula (iv) or (vi), after it is converted to a mixed acid anhydride in accordance with a method described in Step 1, may be reduced with an aqueous sodium borohydride solution in an inert solvent at -20°C to room temperature, preferably at 0°C to give a compound represent by formula (vii).

〈Step 6〉

This is a step of removing an amino-protecting group ($R_A$) of a compound represented by formula (vii). In this step, a customary-used method can be employed. For example, in the case where $R_A$ is a tert-butoxycarbonyl group, it may be carried out as follows.

A compound represented by formula (vii) is subjected to a deprotecting reaction in a solvent such as methanol, ethanol, n-propanol, isopropanol, tetrahydrofuran, 1,4-dioxane, acetic acid or water; or in a mixed solvent of the aforementioned solvents; or in absence of a solvent, in the presence of an acid such as hydrogen chloride, hydrogen bromide, sulfuric acid, formic acid, trifluoroacetic acid or trifluoromethanesulfornic acid or the like, at the temperature from -30 to 70°C, preferably at 0°C to room temperature. As a result, a compound represented by formula (viii) ($R^1$, $R^2$, $R^4$ and <u>m</u>

respectively have the same meanings as defined above) or a salt thereof can be obtained. On the other hand, in the case of using another protecting group, an usual method for removing such the protecting group may be employed.

⟨Step 7⟩

A compound represented by formula (viii) or a salt thereof is allowed to react with an isothiocianate derivative represented by

$R_A$'-NCS

($R_A$' is an amino protecting group) in a solvent such as methanol, ethanol, n-propanaol, isopropanol, tetrahydrofuran, 1,4-dioxane, acetone or a mixed solvent thereof, at the temperature from 0°C to a heating temperature, preferably at room temperature, thereby affording a compound represented by formula (ix) ($R^1$, $R^2$, $R^4$, $R_A$' and m respectively represent the same meanings as defined above).

In this case, when a salt of a compound represented by formula (viii) is used, it is preferred that a base such as triethylamine or N, N-diisopropylethylamine should be further added. And, the protecting group $R_A$' used herein is preferably a protecting group removable under acidic conditions.

⟨Step 8⟩

This is a step of removing an amino-protecting group $R_A$' of a compound represented by formula (ix), and cyclizing the compound, simultaneously. The protecting group can be removed by an usual method. Hereinbelow, we will explain the case where the protecting group $R_A$' is a protecting group removable in acidic conditions, such as a tert-butyl group, by way of example.

A compound represented by formula (ix) is allowed to react in an aqueous solution of acid such as hydrochloric acid, sulfuric acid, and hydrobromic acid in water or an acetic acid solution or the like, preferably in a concentrated hydrochloric acid, concentrated hydrobromic acid, or hydrogen bromide in acetic acid solution; at the temperature from room temperature to reflux temperature, preferably reflux temperature; thereby affording a desired compound represented by formula (I-1) ($R^1$, $R^2$, $R^4$ and m respectively have the same meanings as defined above). On the other hand, in the case of using a protecting group removable in other conditions, the deprotecting step can be made by employing an usual method for removing such the protecting group, and then, cyclizing may be carried out by acid treatment.

When a counter ion of a salt is converted, for example, in the case where a hydrobromide salt is converted to a fumaric acid salt, the hydrobromide salt is dissolved in water and the resultant solution is made into a basic condition using sodium carbonate, potassium carbonate, sodium hydroxide, potassium hydroxide, or the like. The basic solution thus obtained is then subjected to extraction with an organic solvent such as dichloromethane, chloroform, carbon tetrachloride, or ethyl acetate, or the like. Alternatively, partition is performed between the basic solution and the organic solvent. Thereafter, the organic phase is evaporated to dryness and the residue is dissolved in a solvent such as methanol, ethanol, n-propanol or isopropanol or the like. Further, to the resultant solution, addition is made of a solution such as methanol, ethanol, n-propanol, isopropanol or the like in which a desired acid such as fumaric acid is dissolved. As a result, the desired compound with a counter ion converted, represented by formula (I-1) is obtained.

Preparation Method B

The method for producing a compound represented by formula (I) wherein m is 0; p is 1; and $R^3$ and $R^5$ are respectively a hydrogen atom.

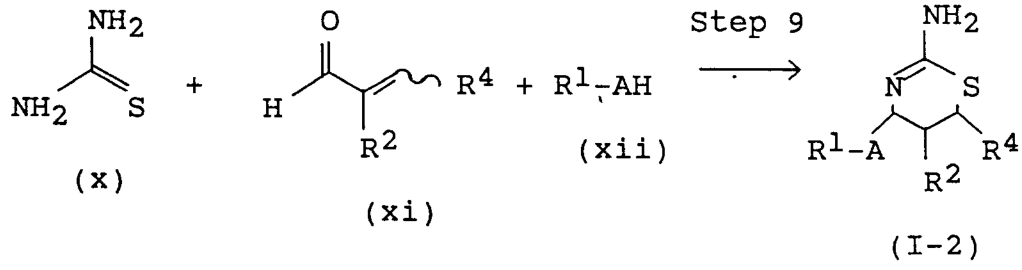

⟨Step 9⟩

In the case where a compound (xii) represented by $R^1$-AH ($R^1$ and A respectively have the same meanings as defined above) is used as a solvent as well as a reagent, thiourea represented by formula (x), together with a known aldehyde compound or a readily preparable compound represented by formula (xi), is allowed to react with the compound (xii) under the action of an acid catalyst such as hydrochloric acid, hyrdobromic acid, or p-toluenesulfonic acid or the like, in the presence or absence of a polymerization inhibiting agent such as hydroquinone, at the temperature from room temperature to reflux temperature, thereby affording a desired compound represented by formula (I-2) ($R^1$, $R^2$, $R^4$ and A respectively have the same meanings as defined above).

When the compound (xii) represented by $R^1$-AH is used as a mere reagent, the same method as described above can be employed except that a solvent such as acetic acid which acts also as an acid catalyst is used, whereby the desired compound represented by formula (I-2) is obtained.

Preparation Method C

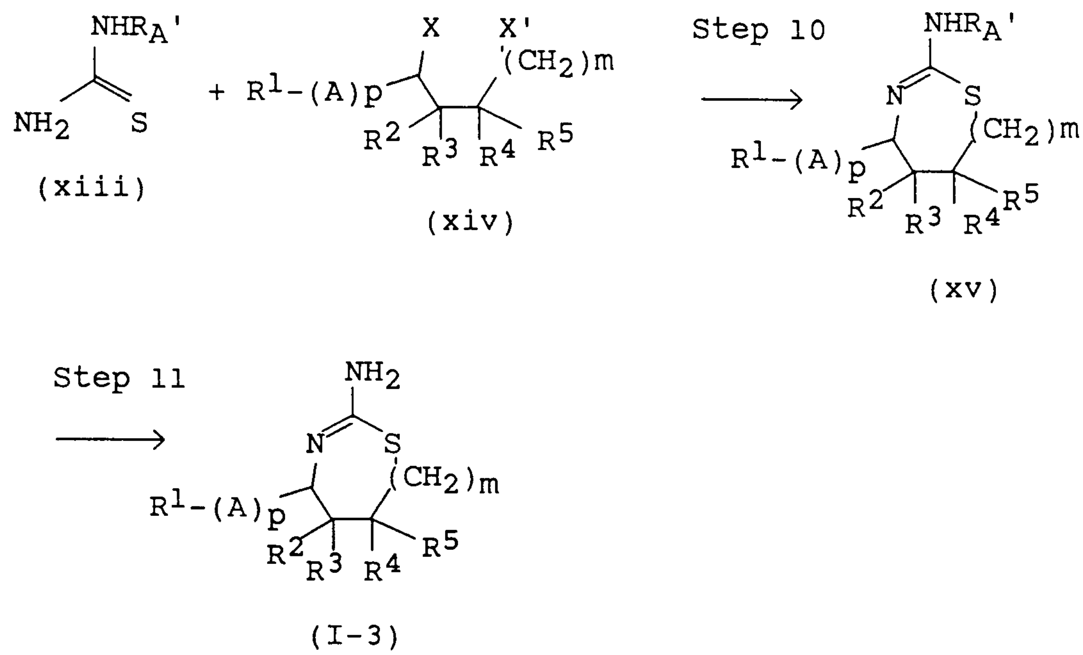


(xiii) (xiv) (xv) (I-3)

⟨Step 10⟩

A compound represented by formula (xiii)($R_A$' has the same meaning as defined above) is allowed to react with a known compound or a compound readily-preparable from a known compound which are represented by formula (xix) (X and X' respectively are a leaving group such as halogen; and $R^1$, $R^2$, $R^3$, $R^4$, $R^5$,A, m and p respectively have the same meaning as defined above), in an inert solvent such as methanol, ethanol, n-propanol, isopropanol, tetrahydrofuran, 1,4-dioxane, or N, N-dimethylformamide or the like, preferably in methanol, ethanol, n-propanol, or isopropanol. In addition, the reaction is carried out in the presence of a base such as sodium hydrogencarbonate, potassium hydrogencarbonate, potassium carbonate, sodium carbonate, triethylamine, N, N-diisopropylethylamine, pyridine, or N-methyl morpholine or the like, at the temperature from room temperature to reflux temperature. As a result, a compound represented by formula (xv) ($R^1$, $R^2$, $R^3$, $R^4$, $R^5$, $R_A$', A, m and p respectively have the same meanings as defined above) is obtained.

⟨Step 11⟩

This is a step of removing an amino-protecting group $R_A$' of a compound represented by formula (xx). An usual method can be employed in this step For example, in the case where $R_A$' is a tert-butyl group, a compound represented by formula (1-3) ($R^1$, $R^2$, $R^3$, $R^4$, $R^5$, A, m and p respectively have the same meanings as defined above) can be obtained

by performing the same reaction as described in Step 8. On the other hand, in the case of using another protection group, an usual method for removing such the protecting group may be employed.

When a compound represented by formula (I-3) in which $R^1$-$(A)_p$- is a hydroxyl group, an amino group, or a thiol group is desired, the removal of a protecting group may be carried out in accordance with an usual method after the similar reaction is performed using a starting compound (xiv) in which $R^1$ is a protecting group.

Preparation Method D

Hereinbelow, we will explain a method of preparing a compound represented by formula (I) in which m is 0, both $R^2$ and $R^3$ are a hydrogen atom.

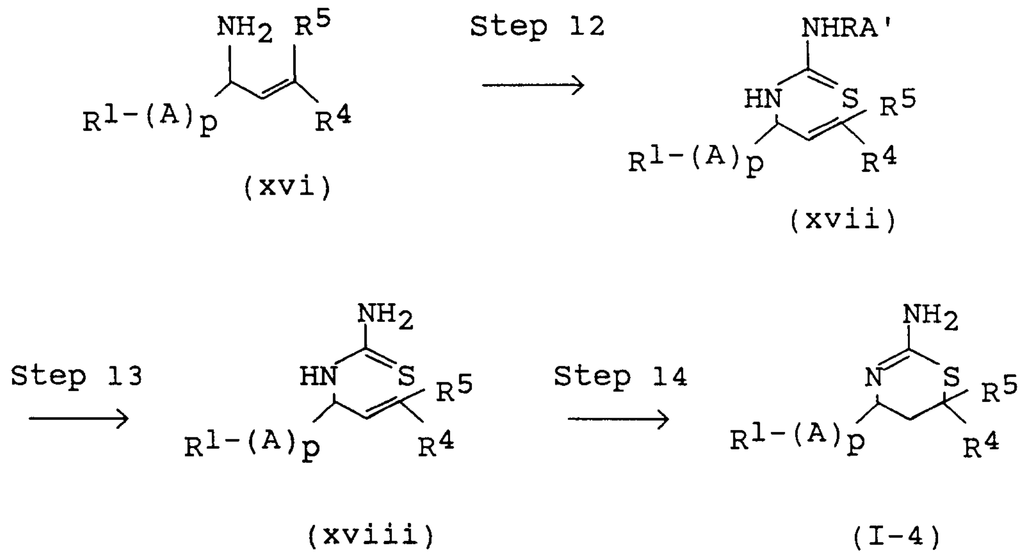


(xvi) (xvii) (xviii) (I-4)

⟨Step 12⟩

Using, as a starting material, a compound represented by formula (xvi) which is known or readily produced in accordance with a known method, the same reaction as described in Step 7 is performed to afford a compound represented by formula (xvii) ($R^1$, $R^4$, $R^5$, $R_A$', A and p respectively have the same meanings as defined above).

⟨Step 13⟩

This is a step to remove an amino-protecting group $R_A$' of a compound represented by formula (xvii). In this step, an usual method can be employed. Hereinbelow, we will explain the case in which the protecting group $R_A$' is that of being removable in basic conditions, such as a benzoyl group.

A compound represented by formula (xvii) is reacted in a mixed solvent between water and a organic solvent such as methanol, ethanol, n-propanol, isopropanol, tetrahydrofuran, or 1,4-dioxane or the like, by using a base such as sodium hydroxide or potassium hydroxide or the like, at the temperature from room temperature to reflux temperature. As a result, a compound represented by formula (xviii)($R^1$, $R^4$, $R^5$, A and p respectively have the same meanings as defined above) can be obtained. On the other hand, in the case of using another protecting group, an usual method for removing such the protecting group may be employed.

⟨Step 14⟩

A compound represented by formula (xviii) is heated under reflux in an aqueous solution of acid such as hydrochloric acid, hydrobromic acid, or sulfuric acid or the like, preferably in an aqueous solution of acid solution such as hydrochloric acid or hydrobromic acid, thereby affording a compound represented by formula (I-4) ($R^1$, $R^4$, $R^5$, A and p respectively have the same meanings as defined above).

Preparation Method E

A compound in which $R^1$-$(A)_p$- is an alkoxyalkyl group such as a methoxymethyl, may be produced in accordance with the aforementioned method. However, by way of reference, a more suitable method will be described below The following method relates to the production of compound (I-5), i.e., a compound represented by formula (I) in which $R^1$-$(A)_p$- is $R_6O(CH_2)_r$ ($R^6$ is an alkyl group, r is an integer from 1 to 6), p=0, m=0, and both $R^4$ and $R^5$ are a hydrogen atom; or compound (I-5'), i.e., a compound represented by formula (I) in which $R^1$-$(A)_p$- is $R_6O(CH_2)_r$ ($R^6$ and r have the same meanings as defined above), p=0, and m=1. Since 2-amino-4,5,6,7-tetrahydro-1,3-thiazepine derivative (I-5') can be produced in the same manner as in the case of 2-amino-5,6-dihydro-4H-1,3-thiazine derivative (I-5) by using a compound represented by formula (xix'), we will describe the method of preparing a 6-membered cyclic compound (I-5), hereinbelow.

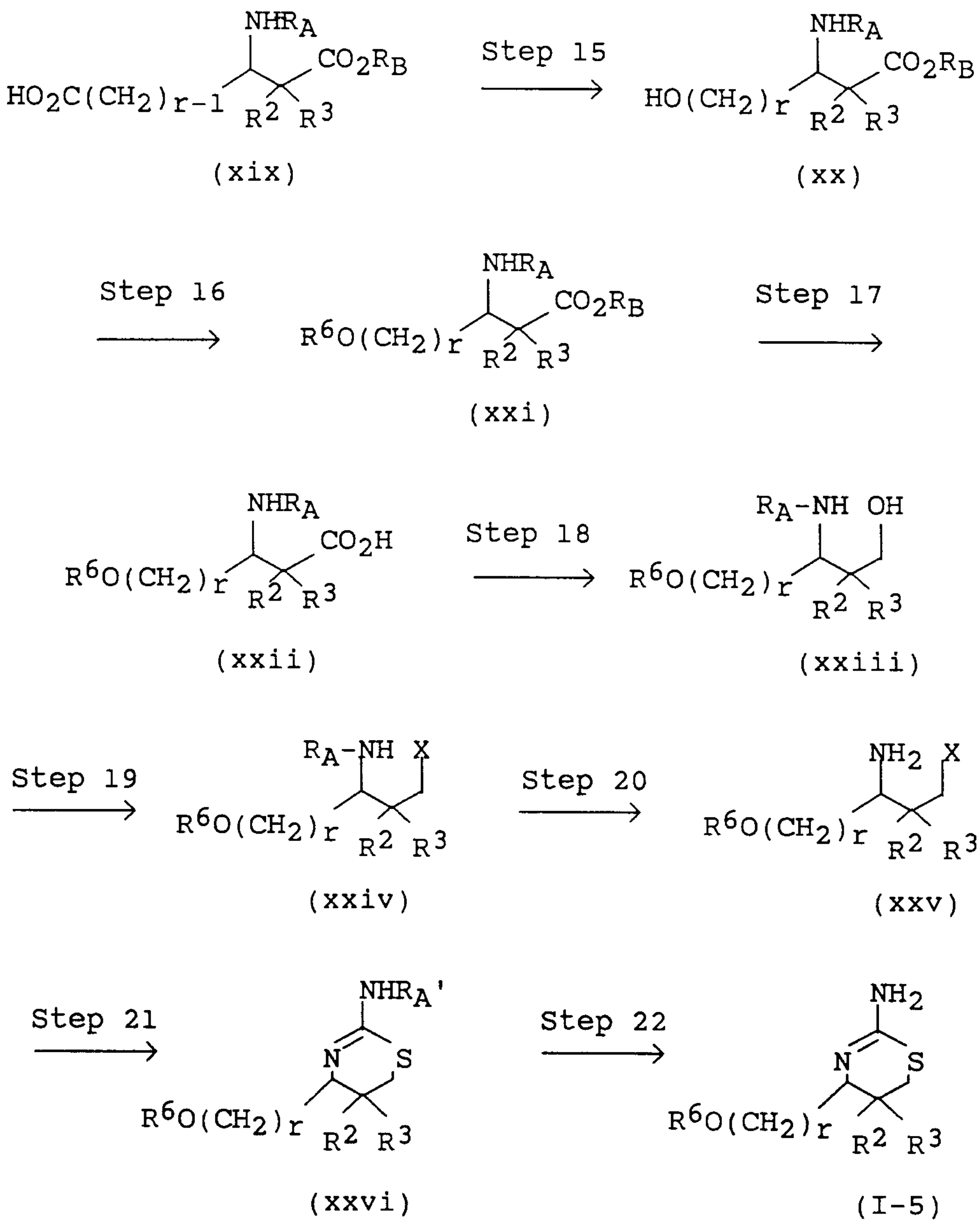


(xix) (xx) (xxi) (xxii) (xxiii) (xxiv) (xxv) (xxvi) (I-5)

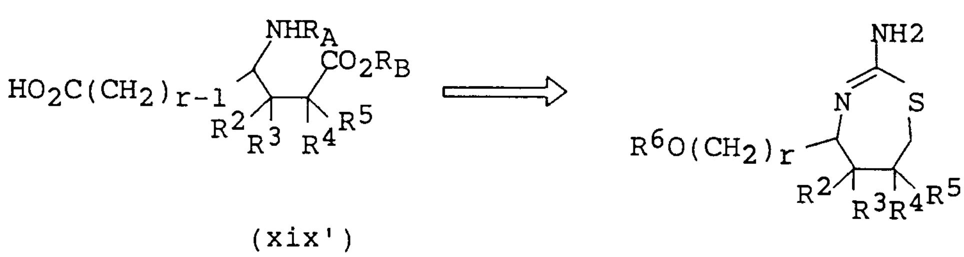

(xix') (I-5')

⟨Step 15⟩

Using compound which is known or compound which is readily produced in accordance with a known method, represented by formula (xix)($R_B$ is a carboxyl-protecting group, $R^2$, $R^3$, $R_A$ and r respectively have the same meanings as defined above) as a starting material, the same reaction described in Step 5 is performed. As a result, a compound represented by formula (xx) ($R^2$, $R^3$, $R_A$, $R_B$ and r respectively have the same meanings as defined above) cab be obtained.

⟨Step 16⟩

A compound represented by formula (xx) is reacted with an alkylating agent such as alkyl halide or dialkylsulfate, in a solvent such as tetrahydrofuran, N,N-dimethylformamide, 1,4-dioxane, acetone, methylethylketone or acetonitrile or the like, in the presence of a base such as sodium hydrogencarbonate, potassium hydrogencarbonate, sodium carbonate, potassium carbonate, silver oxide, triethylamine, N,N-diisopropylethylamine or N-methylmorpholine or the like, thereby affording a compound represented by formula (xxi) ($R^2$, $R^3$, $R^6$, $R_A$, $R_B$ and r respectively have the same meanings as defined above).

⟨Step 17⟩

This is a step to remove a carboxyl-protecting group $R_B$ of a compound represented by general formula (xxi). This step can be carried out by using an usual method For example, in the case where the protecting group $R_B$ is a tert-butyl group, it may be carried out as follows.

A compound represented by formula (xxi) is treated with an acid such as hydrogen chloride, sulfuric acid, hydrogen bromide, trifluoroacetic acid, or trifluoromethane sulfonic acid or the like, in a solvent such as methanol, ethanol, 1,4-dioxane, ethyl acetate, dichloromethane, chloroform, water or a mixed solvent thereof. As a result, a compound represented by formula (xxii) ($R^2$, $R^3$, $R^6$, $R_A$ and r respectively have the same meanings as defined above) can be obtained. On the other hand, in the case of using another protecting group, an usual method for removing such the particular protecting group may be employed.

⟨Step 18⟩

The same reduction reaction as described in Step 5 is applied to a compound represented by formula (xxii), affording a compound represented by formula (xxiii) ($R^2$, $R^3$, $R^6$, $R_A$ and r respectively have the same as defined above).

⟨Step 19⟩

A compound represented by formula (xxiii) is reacted with a halogenating agent such as carbon tetrabromide, N-bromosuccinimide, N-iodesuccinimide, N-chlorosuccinimide, thionyl chloride, or oxalyl chloride or the like in a solvent such as dichloromethane, chloroform, tetrahydrofuran or 1,4-dioxane or the like, in the presence of triphenylphosphine. As a result, a compound represented by formula (xxiv) ($R^2$, $R^3$, $R^6$, $R_A$, X and r respectively have the same meanings as defined above) can be obtained.

⟨Step 20⟩

This is a step to remove an amino-protecting group $R_A$ of a compound represented by formula (xxiv). This step can be carried out by using an usual method. For example, in the case where the protecting group $R_A$ is a benzyloxycarbonyl group, the step may be carried out as follows.

A compound represented by formula (xxiv) is reduced with hydrogen in a solvent such as methanol, ethanol, n-propanol, isopropanol, tetrahydrofuran, 1,4-dioxane, acetic acid, dichloromethane, or N,N-dimethylformamide, or a mixed solvent thereof, in the presence of a catalyst such as palladium-carbon, palladium black, or palladium hydroxide-carbon or the like, at the temperature from room temperature to 60°C, preferably at room temperature. As a result, a compound represented by formula (xxv) ($R^2$, $R^3$, $R^6$, X and r respectively have the same meanings as defined above) can be obtained. Alternatively, acid treatment may be carried out to remove a protecting group, as described in Step 8. On the other hand, in the case of using another protecting group, the deprotecting step may be performed by using an usual method for removing such the protecting group.

⟨Step 21⟩

A compound represented by formula (xxv) or a salt thereof is reacted with an isothiocyanate derivative represented by $R_A$'-NCS ($R_A$' is the same as defined above), in methanol, ethanol, n-propanol, isopropanol, tetrahydrofuran, 1,4-dioxane, acetone, or a mixed solvent thereof, at the temperature from 0°C to a heating temperature, preferably at the temperature from room temperature to heating temperature. As a result, a compound represented by formula (xxvi) ($R^2$, $R^3$, $R^6$ and r respectively have the same meanings as defined above) can be obtained.

⟨Step 22⟩

This is a step to remove an amino-protecting group $R_A$' of a compound represented by formula (xxvi). An usual method can be employed in this step In the case where the protecting group $R_A$' is, for example, a tert-butyl group, the same method described in Step 11 can be applied thereto. As a result, a compound represented by formula (I-5)($R^2$, $R^3$, $R^6$ and r respectively have the same meanings as defined above) can be obtained.

Hereinbelow, the compound of the present invention represented by formula (I) and the preparation method thereof will be explained more specifically by way of Examples. However, the present invention will not be limited by the Examples.

Example 1 (Preparation Method A)

(S)-2-amino-5,6-dihydro-4-isopropyl-4H-1,3-thiazine hydrobromide

⟨1⟩: Step 1

(R)-3-tert-butoxycarbonylamino-1-diazo-4-methyl-2-pentanone

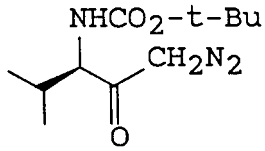

To a tetrahydrofuran solution (200 ml) of N-tert-butoxycarbonyl-D-valine (15.0 g), triethylamine (13 ml) and ethyl chlorocarbonate (7.9 ml) were added at -20°C. The resultant mixture was stirred for 1 hour, added with diazomethane (0.7M diethyl ether solution: 200 ml), and then, further stirred overnight. After the reaction mixture was concentrated, the residue was dissolved in ethyl acetate and washed successively with a saturated aqueous solution of sodium hydrogencarbonate and an aqueous sodium chloride solution. The ethyl acetate layer was dried over anhydrous sodium sulfate and then concentrated, affording the title compound (17.3 g).

⟨2⟩: Step 2

(S)-3-tert-butoxycarbonylamino-4-methyl-pentanoic acid

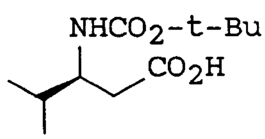

(R)-3-tert-butoxycarbonylamino-1-diazo-4-methyl-2-pentanone (17.3 g) obtained in the aforementioned ⟨1⟩ was dissolved in a mixture of water (100 ml) and 1,4-dioxane (150 ml). To the resultant solution, silver benzoate (10 % solution in triethylamine 5 ml;) was added over 3 hours, while stirring at 50°C. After the reaction mixture was filtrated, it was concentrated under reduced pressure and the residue was dissolved in ethyl acetate. The ethyl acetate layer was

extracted with a saturated aqueous solution of sodium hydrogencarbonate. To the aqueous layer, potassium hydrogensulfate was added to make the solution into acidic condition. To this acidic solution, ethyl acetate was added and allowed to separate. The ethyl acetate layer was obtained and washed with water. The resultant ethyl acetate layer was dried over anhydrous sodium sulfate and concentrated, affording the title compound (13.8 g).

⟨3⟩: Step 5

(S)-3-tert-butoxycarbonylamino-4-methyl pentanol

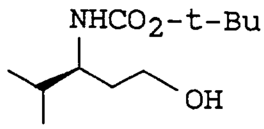

(S)-3-tert-butoxycarbonylamino-4-methylpentanoic acid (13.8 g) obtained in the aforementioned ⟨2⟩ was dissolved in tetrahydrofuran (65 ml). After borane-tetrahydrofuran complex (1M tetrahydrofuran solution: 65 ml) was added thereto at -18°C, the temperature of the reaction mixture was warmed to room temperature and the mixture was stirred for 1 hour. After the reaction mixture was cooled to 0°C, water (20 ml) was added thereto to terminate the reaction and then concentrated. The concentrate was dissolved in ethyl acetate and washed successively with an aqueous potassium hydrogensulfate solution, an aqueous sodium hydrogencarbonate solution, and water. The ethyl acetate layer was dried over anhydrous sodium sulfate and concentrated, affording the title compound (5.58 g).

⟨4⟩: Step 6

(S)-3-amino-4-methyl-pentanol hydrochloride

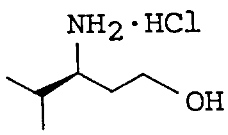

To (S)-3-tert-butoxycarbonylamino-4-methylpentanol (5.58 g) obtained in the aforementioned ⟨3⟩, 4N hydrogen chloride-1,4-dioxane solution was added. The resultant mixture was stirred for 30 minutes at room temperature and concentrated, affording the title compound (4.72 g).

⟨5⟩: Step 7

(S)-3-(3-tert-butylthioureido-4-methylpentanol

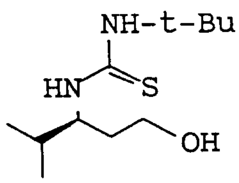

To an ethanol solution (25 ml) of (S)-3-amino-4-methylpentanol hydrochloride obtained in the aforementioned ⟨4⟩, triethylamine (4.0 ml) and tert-butyl isothiocyanate (4.0 ml) were added. The resultant mixture was stirred for 19 hours at room temperature. After concentration of the reaction mixture, the residue was dissolved in ethyl acetate, and washed successively with an aqueous potassium hydrogensulfate solution, an aqueous sodium hydrogencarbonate solution and a saturated aqueous solution of sodium chloride. The ethyl acetate layer thus obtained was dried over anhydrous sodium sulfate and concentrated, affording the title compound (2.2 g).

⟨6⟩: Step 8

(S)-2-amino-5,6-dihydro-4-isopropyl-4H-1,3-thiazine hydrobromide

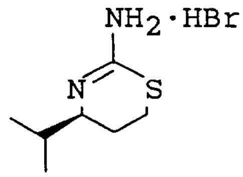

To (S)-3-(3-tert-butylthioureido-4-methylpentanol (2.2 g) obtained in the aforementioned ⟨5⟩, concentrated hydrobromic (15 ml) was added and heated under reflux for 3 hours. The reaction mixture was concentrated, affording the title compound (2.1 g) as a white crystal.

Melting point: 151°C

$^1H$ NMR (300 MHz, δ ppm, DMSO-$d_6$): 0.92 (3H, d, J=7.0 Hz), 0.95 (3H, d, J=7.0Hz), 1.73-1.93 (2H, m), 2.14 (1H, m), 3.16-3.23 (2H, m), 3.41 (1H, m), 8.70 (2H, broad s), 9.50 (1H, broad s)

FAB (+) MS (low resolution): 159.1

$[\alpha]_D$: -11.2 (c=1.30, $H_2O$)

Example 2 (Preparation Method A)

(R)-2-amino-5,6-dihydro-4-isopropyl-4H-1,3-thiazine hydrobromide

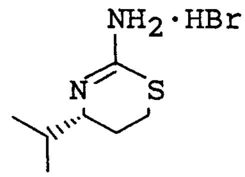

The title compound (3.75 g) was obtained as a white crystal starting from N-tert-butoxycarbonyl-L-valine (15.0 g), in the same manner as in Example 1.

Melting point: 151 - 152°C

$^1H$ NMR (300 MHz, δppm, $CDCl_3$): 1.07 (3H, d, J=7.0 Hz), 1.09 (3H, d, J=7.0 Hz), 1.91-2.06 (2H, m), 2.25 (1H, m), 3.17-3.33 (2H, m), 3.43 (1H, m), 8.50 (2H, broad s), 9.76 (1H, broad s)

FAB (+) MS (low resolution): 159.1

$[\alpha]_D$: +11.1 (c=1.04, $H_2O$)

Example 3 (Preparation Method A)

(R)-2-amino-5,6-dihydro-4-propyl-4H-1,3-thiazine hydrobromide

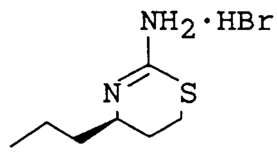

The title compound (1.70 g) was obtained as a white crystal starting from N-tert-butoxycarbonyl-D-norvaline (25.0 g), in the same manner as in Example 1.

Melting point: 108 - 109°C

$^1$H NMR (300 MHz, δppm, DMSO-$d_6$): 0.89 (3H, t, J=6.0 Hz), 1.25-1.65 (4H, m), 1.65-1.85 (1H, m), 2.10-2.25 (1H, m), 3.15-3.25 (2H, m), 3.50-3.60 (1H, m), 8.65 (2H, broad s), 9.59 (1H, s)

FAB (+) MS (low resolution): 159.0

$[\alpha]_D$: +34.2 (c=0.84, $H_2O$)

Example 4 (Preparation Method A)

(S)-2-amino-5,6-dihydro-4-propyl-4H-1,3-thiazine hydrobromide

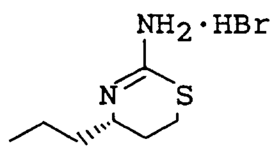

The title compound (5.0 g) was obtained as a white crystal starting from N-tert-butoxycarbonyl-L-norvaline (15.18 g), in the same manner as in Example 1.

Melting point: 108 - 109°C

$^1$H NMR (300 MHz, δppm, DMSO-$d_6$):0.89 (3H, t, J=6.0 Hz), 1.31-1.60 (4H, m), 1.69-1.81 (1H, m), 2.11-2.21 (1H, m), 3.17-3.24 (2H, m), 3.54(1H, m), 8.60 (2H, broad s), 9.61 (1H, s)

FAB (+) MS (low resolution): 159.1

$[\alpha]_D$: -34.6 (c=1.00, $H_2O$)

Example 5 (Preparation Method A)

(R)-2-amino-4-butyl-5,6-dihydro-4H-1,3-thiazine hydrobromide

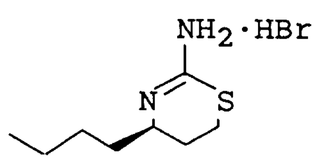

The title compound (2.01 g) was obtained as a white crystal starting from N-tert-butoxycarbonyl-D-norleucine dicyclohexylamine salt (25.0 g), in the same manner as in Example 1.

Melting point: 155 - 156°C

$^1H$ NMR (300 MHz, δppm, DMSO-$d_6$): 0.90 (3H, t, J=6.0 Hz), 1.20-1.40 (4H, m), 1.45-1.65 (2H, m), 1.70-1.85 (1H, m), 2.10-2.25 (1H, m), 3.15-3.25 (2H, m), 3.45-3.65 (1H, m), 8.60 (2H, broad s), 9.63 (1H, s)

FAB (+) MS (low resolution): 173.1

$[\alpha]_D$: +32.8 (c=0.89, $H_2O$)

Example 6 (Preparation Method A)

(S)-2-amino-4-butyl-5,6-dihydro-4H-1,3-thiazine hydrobromide

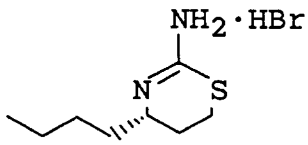

The title compound (0.99 g) was obtained as a white crystal starting from N-tert-butoxycarbonyl-L-norleucine (15.0 g), in the same manner as in Example 1.

Melting point: 155 - 156°C

$^1H$ NMR (300 MHz, δppm, DMSO-$d_6$): 0.89 (3H, t, J=6.0 Hz), 1.20-1.40 (4H, m), 1.45-1.70 (2H, m), 1.75-1.85 (1H, m), 2.10-2.25 (1H, m), 3.15-3.25 (2H, m), 3.50-3.65 (1H, m), 8.65 (2H, broad s), 9.65 (1H, s)

FAB (+) MS (low resolution): 173.1

$[\alpha]_D$: -33.7 (c=1.00, $H_2O$)

Example 7 (Preparation Method A)

(R)-2-amino-5,6-dihydro-4-isobutyl-4H-1,3-thiazine hydrobromide

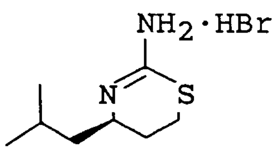

The title compound (1.20 g) was obtained as a white crystal starting from N-tert-butoxycarbonyl-D-leucine (15.0 g), in the same manner as in Example 1.

Melting point: 121 - 122°C

$^1H$ NMR (300 MHz, δppm, DMSO-$d_6$): 0.95(3H, t, J=6.2 Hz), 0.98 (3H, d, J=6.2Hz), 1.43 (1H, m), 1.69 (1H, m), 1.82-1.99 (2H, m), 2.31 (1H, m), 3.16-3.33 (2H, m), 3.66 (1H, m), 8.50 (2H, broad s), 9.78 (1H, s)

FAB (+) MS (low resolution): 172.7

$[\alpha]_D$: +41.7 (c=1.01, $H_2O$)

Example 8 (Preparation Method A)

(S)-2-amino-5,6-dihydro-4-isobutyl-4H-1,3-thiazine hydrobromide

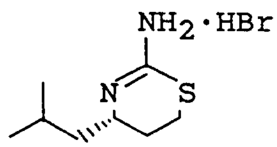

The title compound (1.80 g) was obtained as a white crystal starting from N-tert-butoxycarbonyl-L-leucine (15.0 g), in the same manner as in Example 1.

Melting point: 117 - 119°C

$^1$H NMR (300 MHz, δppm, DMSO-$d_6$): 0.95(3H, t, J=6.2 Hz), 0.98 (3H, d, J=6.2Hz), 1.42 (1H, m), 1.69 (1H, m), 1.82-1.99 (2H, m), 2.31 (1H, m), 3.16-3.33 (2H, m), 3.66 (1H, m), 8.48 (2H, broad s), 9.81 (1H, s)

FAB (+) MS (low resolution): 172.9

$[\alpha]_D$: -40.4 (c=0.77, $H_2O$)

Example 9 (Preparation Method A)

(R)-2-amino-4-benzyl-5,6-dihydro-4H-1,3-thiazine hydrobromide

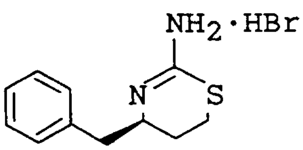

The title compound (1.62 g) was obtained as a white crystal starting from N-tert-butoxycarbonyl-D-phenylalanine (15.0 g), in the same manner as in Example 1.

Melting point: 216- 217°C

$^1$H NMR (300 MHz, δppm, DMSO-$d_6$): 1.06(1H, m), 1.90-2.10 (1H, m), 2.75-2.90 (1H, m), 2.95-3.05 (1H, m), 3.10-3.25 (2H, m), 3.80-3.95 (1H, m), 7.20-7.40 (5H, m), 8.65 (2H, broad s), 9.62 (1H, s)

FAB (+) MS (low resolution): 206.8

$[\alpha]_D$: +40.9 (c=1.235, $H_2O$)

Example 10 (Preparation Method A)

(S)-2-amino-4-benzyl-5,6-dihydro-4H-1,3-thiazine hydrobromide

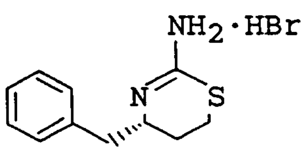

The title compound (1.79 g) was obtained as a white crystal starting from N-tert-butoxycarbonyl-L-phenylalanine (15.0 g), in the same manner as in Example 1.

Melting point: 215 - 217°C

$^1H$ NMR (300 MHz, δ ppm, DMSO-$d_6$): 1.06-1.08 (1H, m), 2.00-2.15 (1H, m), 2.80-2.90 (1H, m), 2.95-3.05 (1H, m), 3.10-3.25 (2H, m), 3.80-3.95 (1H, m), 7.20-7.40 (5H, m), 8.65 (2H, broad s), 9.65 (1H, s)

FAB (+) MS (low resolution): 206.7

$[\alpha]_D$: -43.1 (c=0.94, $H_2O$)

Example 11 (Preparation Method A)

(S)-2-amino-4-(3-aminopropyl)-5,6-dihydro-4H-1,3-thiazine dihydrobromide

⟨1⟩: Steps 1, 2 and 5

(S)-3-benzyloxycarbonylamino-6-tert-butoxycarbonylaminohexanol

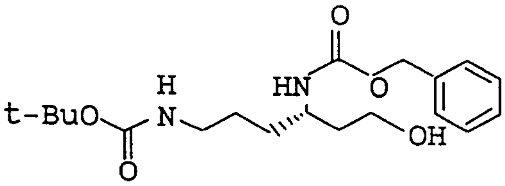

(S)-5-amino-2-benzyloxycarbonylaminopentanoic acid was protected with a tert-butoxycarbonyl group in accordance with a known method. From the thus obtained (S)-2-benzyloxycarbonylamino-5-tert-butoxycarbonylaminopentanoic acid (33.86 g), the title compound (13.16 g) was obtained in the same manner as previously described ⟨1⟩, ⟨2⟩ and ⟨3⟩ in Example 1.

⟨2⟩: Steps 6 and 7

(S)-6-tert-butoxycarbonylamino-3-(3-tert-butylthioureido)hexanol

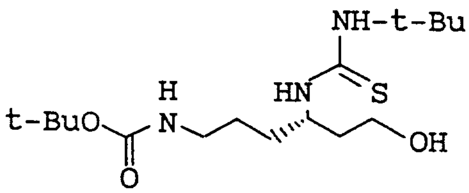

To an ethanol solution (100 ml) of (S)-3-benzyloxycarbonylamino-6-tert-butoxycarbonylaminohexanol (13.16 g) obtained in the aforementioned ⟨1⟩, 10% palladium carbon (1.31 g) was added. The resultant mixture was stirred at room temperature for 5.5 hours under a hydrogen atmosphere. After the catalyst was filtered off, tert-butyl isothiocyanate (5.20 ml) was added to the filtrate and the resultant mixture was stirred for 51 hours at room temperature. The reaction mixture was concentrated, dissolved in ethyl acetate, and then washed successively with an aqueous potassium hydrogensulfate, an aqueous sodium hydrogencarbonate solution, and a saturated aqueous solution of sodium chloride. The ethyl acetate layer was dried over anhydrous sodium sulfate and concentrated, affording the title compound (11.47 g).

⟨3⟩: Step 8

(S)-2-amino-4-(3-aminopropyl)-5,6-dihydro-4H-1,3-thiazine) dihydrobromide

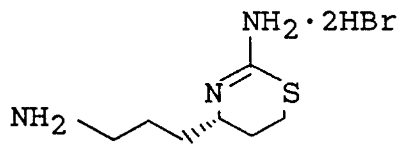

The title compound (1.83 g) was obtained as a white crystal starting from (S)-6-tert-butoxycarbonylamino-3-(3-tert-butylthioureido)hexanol (3.00 g) obtained in the aforementioned ⟨2⟩, in the same manner as previously mentioned ⟨6⟩ in Example 1.

Melting point: 124 - 128°C

$^1$H NMR (300MHz, δppm, DMSO-$d_6$): 1.50-1.70 (4H, m), 1.70-1.85 (1H, m), 2.05-2.20 (1H, m), 2.70-2.90 (2H, m), 3.15-3.25 (2H, m), 3.55-3.70 (1H, m), 7.85 (3H, broad s), 8.68 (2H, broad s), 9.75 (1H,s)

FAB (+) MS (low resolution): 174.1

$[\alpha]_D$: -19.8 (c=1.04, $H_2O$)

Example 12 (Preparation Method A)

Bis(2-amino-4,5,6,7-tetrahydro-4-methyl-1,3-thiazepine) fumarate

⟨1⟩: Steps 3 and 4

4-tert-butoxycarbonylaminopentanoic acid

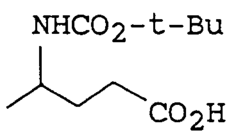

The title compound (20.58 g) was obtained starting from 3-tert-butoxycarbonylaminobutyric acid (24.39 g), in the same manner as previously mentioned ⟨1⟩ and ⟨2⟩ in Example 1.

⟨2⟩: Step 5

4-tert-butoxycarbonylaminopentanol

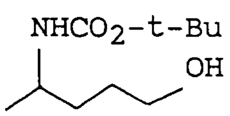

To a tetrahydrofuran solution (200 ml) of 4-tert-butoxycarbonylaminopentanoic acid (20.58 g) obtained in aforementioned ⟨1⟩, triethylamine (14.0 ml) was added. To the resultant mixture, ethyl chlorocarbonate (10.0 ml) was further added while maintaining the temperature at -15°C and the reaction solution was stirred for 1 hour. The insoluble material in the reaction solution was filtrated off and the filtrate was cooled at 0°C. To the filtrate, an aqueous solution (80 ml) of sodium borohydride (7.94 g) was added and the resultant mixture was stirred for 1 hour. To the reaction mixture, a

saturated aqueous solution of potassium hydrogensulfate (30 ml) was added to terminate the reaction, followed by concentration. The residue was dissolved in ethyl acetate and washed successively with an aqueous potassium hydrogensulfate, an aqueous sodium hydrogencarbonate solution, and water. The ethyl acetate layer was dried over anhydrous sodium sulfate and concentrated, affording the title compound (13.09 g).

⟨3⟩: Steps 6, 7 and 8

2-amino-4,5,6,7-tetrahydro-4-methyl-1,3-thiazepine hydrobromide

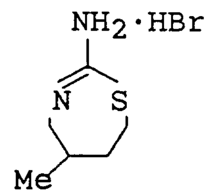

The title compound (2.6 g) was obtained starting from 4-tert-butoxycarbonylaminopentanol (13.09 g) obtained in the aforementioned ⟨2⟩, in the same manner as previously described ⟨4⟩, ⟨5⟩ and ⟨6⟩ in Example 1.

⟨4⟩: Step 8

Bis(2-amino-4,5,6,7-tetrahydro-4-methyl-1,3-thiazepine), fumarate

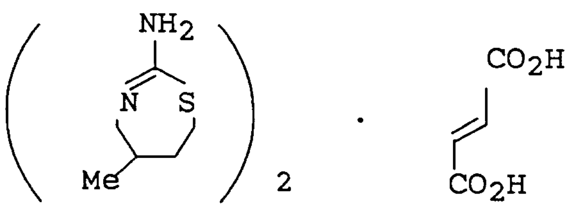

2-amino-4,5,6,7-tetrahydro-4-methyl-1,3-thiazepine hydrobromide (2.6 g) obtained in the aforementioned ⟨3⟩ was partitioned between chloroform and an aqueous potassium carbonate solution. The chloroform fraction was washed with a saturated aqueous solution of sodium chloride. The resultant organic layer was dried over anhydrous sodium sulfate and concentrated. The residue thus obtained was dissolved in 2-propanol, and then, fumaric acid (0.62 g) was added thereto and dissolved with heating. After allowed to stand in a refrigerator for overnight, the crystallized product was recovered by filtration and dried, affording the title compound (1.26 g) as a white crystal.

Melting point: 142 - 145°C

$^1$H NMR (300 MHz, δppm, $D_2O$): 1.17 (6H, d, 6.0 Hz), 1.67 (8H, m), 3.07 (4H, m), 3.78 (2H, m), 6.49 (2H, s)

FAB (+) MS (low resolution): 145

Example 13 (Preparation Method A)

(R)-2-amino-4,5,6,7-tetrahydro-4-propyl-1,3-thiazepine hydrobromide

⟨1⟩: Steps 1 and 2

(R)-3-tert-butoxycarbonylaminohexanoic acid

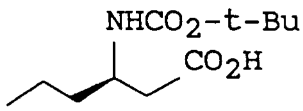

The title compound (15.48 g) was obtained starting from N-tert-butoxycarbonyl-D-norvaline (20.20 g), in the same manner as previously described ⟨1⟩ and ⟨2⟩ in Example 1.

⟨2⟩: Steps 3 and 4

(R)-2-amino-4,5,6,7-tetrahydro-4-propyl-1,3-thiazepine hydrobromide

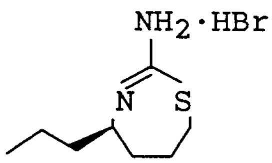

The title compound (1.30 g) was obtained as a white crystal starting from (R)-3-tert-butoxycarbonylamino-hexanoic acid (15.48 g), in the same manner as previously described ⟨1⟩, ⟨2⟩ and ⟨3⟩ in Example 12.

Melting point: 151 - 15°C

$^1$H NMR (300 MHz, δppm, DMSO-$d_6$): 0.87 (3H, t, J=6.0 Hz), 1.26-1.56 (4H, m), 1.59-1.70 (1H, m), 1.75-1.82 (1H, m), 1.87-2.02 (2H, m), 3.01-3.09 (1H, m), 3.13-3.20 (1H, m), 3.60-3.72 (1H, m), 8.58 (1H, broad s), 9.39 (1H, broad s), 9.48 (1H, d, J=3.0 Hz)

FAB (+) MS (low resolution): 173.0

$[\alpha]_D$: +184.9(c=1.03, $H_2O$)

Example 14 (Preparation Method A)

(S)-2-amino-4,5,6,7-tetrahydro-4-propyl-1,3-thiazepine hydrobromide

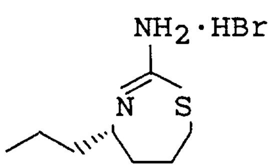

The title compound (1.07 g) was obtained as a white crystal starting from N-tert-butoxycarbonyl-L-valine (30.0 g), in the same manner as in Example 13.

Melting point: 153°C

$^1H$ NMR (300 MHz, δppm, DMSO-$d_6$): 0.87 (3H, t, J=6.0 Hz), 1.28-1.55 (4H, m), 1.59-1.68 (1H, m), 1.75-1.82 (1H, m), 1.87-2.02 (2H, m), 3.01-3.09 (1H, m), 3.13-3.20 (1H, m), 3.60-3.72 (1H, m), 8.58 (1H, broad s), 9.40 (1H, broad, s), 9.48 (1H, d, J=3.0Hz)

FAB (+) MS (low resolution): 173.1

$[\alpha]_D$: -166.6 (c=1.01, $H_2O$)

Example 15 (Preparation Method A)

(R)-2-amino-4-benzyl-4,5,6,7-tetrahydro-1,3-thiazepine hydrobromide

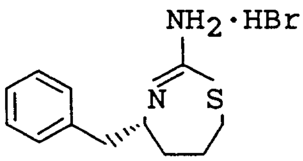

The title compound (0.70 g) was obtained as a white crystal starting from N-tert-butoxycarbonyl-L-phenylalanine (30.0 g), in the same manner as in Example 13.

Melting point: 210 - 211°C

$^1H$ NMR (300 MHz, δ ppm, DMSO-$d_6$): 1.50-1.60 (1H, m), 1.78-1.93 (2H, m), 1.97-2.04 (1H, m), 2.86 (1H, dd, J=4.9 and 10.5 Hz), 3.00-3.11 (2H, m), 3.17-3.23 (1H, m), 3.98-4.07 (1H, m), 7.21-7.32 (5H, m), 8.60 (1H, broad s), 9.39 (1H, broad, s), 9.58 (1H, d, J=3.8 Hz)

FAB (+) MS (low resolution): 211.0

$[\alpha]_D$: -122.5 (c=0.88, $H_2O$)

Example 16 (Preparation Method A, Steps 7 and 8)

2-amino-5,6-dihydro-5,5-dimethyl-4H-1,3-thiazine hydrobromide

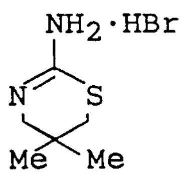

To an ethanol solution (50 ml) of a known 3-amino-2,2-dimethyl-1-propanol (5.15 g), tert-butyl isothiocyanate (6.03 ml) was added. The resultant mixture was stirred for 101 hours at room temperature, and then concentrated. To the oily product thus obtained, concentrated hydrobromic acid (30 ml) was added and heated under reflux for 6 hours. The reaction mixture was concentrated, affording the title compound (4.2 g) as a white crystal.

$^1H$ NMR (300 MHz, δppm, DMSO-$d_6$): 1.04 (6H, s), 3.00 (2H, m), 3.11 (2H, m), 8.75 (2H, broad s), 9.66 (1H, s)

FAB (+) MS (low resolution): 144.7

Example 17 (Preparation Method B)

2-amino-5,6-dihydro-4-hydroxyl-4H-1,3-thiazine hydrochloride

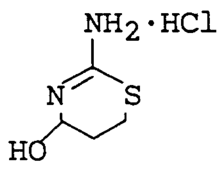

To an aqueous solution (60 ml) of thiourea (4.0 g), acrolein (4.44 ml) and concentrated hydrochloric acid (4.6 ml) were added. The resultant mixture was heated under reflux for 1.5 hours. After cooling, the reaction mixture was washed with chloroform. The water fraction was concentrated, affording the title compound (5.0 g) as a white crystal.

Melting point: 128°C

$^1$H NMR (300MHz, δppm, DMSO-$d_6$): 1.86-1.97 (1H, m), 2.02-2.12 (1H, m), 3.06-3.14(1H, m), 3.24-3.33 (1H, m), 5.09 (1H, broad s), 6.86 (1H, broad, s), 8.96 (1H, broad s), 9.32 (1H, broad s), 10.43 (1H, broad s)

FAB (+) MS (low resolution): 132.7, 114.4

Example 18 (Preparation Method B)

Bis(2-amino-5,6-dihydro-4-methoxy-4H-1,3-thiazine) fumarate

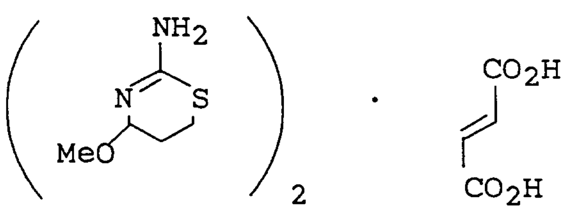

The reaction was performed in the same manner as in Example 17 except that methanol was used instead of water and that the heating under reflux was performed for 8 hours. The reaction mixture was concentrated and the concentrate was partitioned between chloroform and an aqueous ammonia. The chloroform layer thus obtained was washed with a saturated aqueous solution of sodium chloride, dried over anhydrous sodium sulfate, and concentrated under reduced pressure. The residue was dissolved in isopropanol. To the resultant solution, fumaric acid was added, heated to dissolve, and allowed to stand in a refrigerator, affording the title compound (0.11 g) as a white crystal.

$^1$H NMR (300 MHz, δppm, DMSO-$d_6$): 1.88-1.90 (4H, m), 2.98-3.04 (2H, m), 3.07-3.13 (2H, m), 3.32 (6H, s), 4.59-4.62 (2H, m), 6.44 (2H,s)

FAB (+) MS (low resolution): 146.7, 114.4

Example 19 (Preparation Method B)

2-amino-5,6-dihydro-4-propyloxy-4H-1,3-thiazine hydrochloride

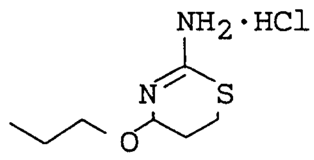

To n-propanol (100 ml), concentrated hydrochloric acid (2.75 ml), hydroquinone (35 mg), acrolein (2.45 ml) and thiourea (2.28 g) were successively added. The resultant mixture was stirred for 4 hours at 70°C and then concentrated. To the concentrate, isopropanol and diethy lether were added, affording the title compound (4.0 g) as a white crystal.

$^1H$ NMR (300 MHz, δppm, DMSO-$d_6$): 0.85(3H, t, J=6.0 Hz), 1.50 (2H, m), 1.88 (1H, m), 2.27 (1H, m), 3.08 (1H, m), 3.25 (1H, m), 3.44 (1H, m), 3.56 (1H, m), 4.91 (1H, m), 9.02 (1H, broad s), 9.60 (1H, broad, s), 11.12 (1H, broad s),

FAB (+) MS (low resolution): 146.7, 114.4

Example 20 (Preparation Method B)

2-amino-5,6-dihydro-4-isopropoxy-4H-1,3-thiazine fumarate

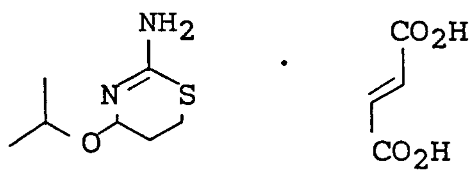

The title compound (0.75 g) was obtained as a white crystal in the same manner as in Example 18 except that isopropanol was used instead of methanol.

Melting point: 145°C

$^1H$ NMR (300 MHz, δppm, DMSO-$d_6$): 1.12 (6H, m), 1.85 (1H, m), 2.10 (1H, m), 3.04 (1H, m), 3.19 (1H, m), 3.91 (1H, m), 4.93 (1H, t, J=3.0Hz), 6.47 (2H, s)

FAB (+) MS (low resolution): 174

Example 21 (Preparation Method B)

Bis(2-amino-4-ethoxy-5,6-dihydro-4H-1,3-thiazine) fumarate

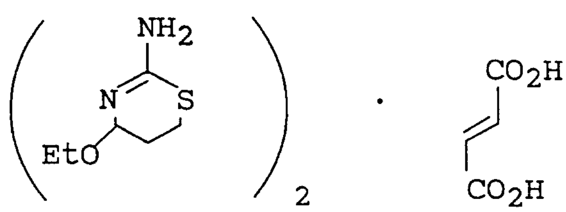

The title compound (3.0 g) was obtained as a white crystal in the same manner as in Example 18 except that ethanol was used instead of methanol.

Melting point: 136 - 137°C

$^1$H NMR (300 MHz, δppm, DMSO-$d_6$): 1.11 (6H, t, J=7.0 Hz), 1.87 (4H, m), 3.01 (2H, m), 3.13 (2H, m), 3.45 (2H, m), 3.73 (2H, m), 4.72 (2H, t, J=3.5 Hz), 6.42 (2H, s)

FAB (+) MS (low resolution): 160.0

<u>Example 22 (Preparation Method B)</u>

Bis(2-amino-4-benzyloxy-5,6-dihydro-4H-1,3-thiazine) fumarate

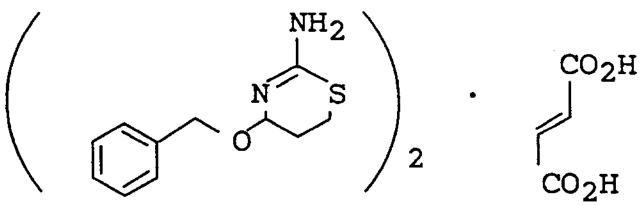

To acetic acid (30 ml), thiourea (2.28 g) and benzyl alcohol (3.25 ml) were added, and then, acrolein (2.1 ml) was further added thereto under a nitrogen atmosphere. After stirred for overnight at 50°C, the reaction mixture was concentrated. To the residue, a saturated solution of sodium hydrogencarbonate was added to give a basic condition. After this mixture was extracted with chloroform, the chloroform layer was washed with a saturated aqueous solution of sodium chloride, dried over anhydrous sodium sulfate and concentrated. The residue was purified by silica gel column chromatography (eluent: chloroform:methanol/100:1 to 20:1). The free base corresponding to the title compound was dissolved in isopropanol. To the resultant solution, fumaric acid (0.74 g) was added and dissolved with heating, and thereafter, the reaction mixture was allowed to stand for overnight in a refrigerator. The crystallized product was recovered and dried, affording the title compound (1.82 g) as a white crystal.

Melting point: 123°C

$^1$H NMR (300 MHz, δppm, DMSO-$d_6$): 1.93 (4H, m), 3.01 (2H, m), 3.15 (2H, m), 4.55 (2H, d, J=12.0Hz), 4.75 (2H, d, J=12.0Hz), 4.81 (2H, m), 6.48 (2H, s), 7.32 (10H, m)

FAB (+) MS (low resolution): 222

<u>Example 23 (Preparation Method B)</u>

Bis(2-amino-5,6-dihydro-4-cyclopropylmethoxy-4H-1,3-thiazine) fumaric acid salt

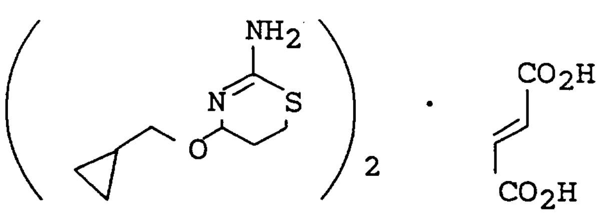

The title compound (1.7 g) was obtained as a white crystal in the same manner as in Example 22 except that cyclopropanemethanol was used instead of benzyl alcohol.

Melting point: 133 - 135°C

$^1$H NMR (300 MHz, δppm, $D_2O$): 0.17 (4H, m), 0.50 (4H, m), 1.01 (2H, m), 1.99 (2H, m), 2.41 (2H, m), 3.04 (2H, m), 3.31-3.49 (6H, m), 5.01 (2H, t, J=3.0 Hz), 6.45 (2H, s)

FAB (+) MS (low resolution): 132.7, 114.4

Example 24 (Preparation Method B)

Bis(4-allyloxy-2-amino-5,6-dihydro-4H-1,3-thiazine) fumarate

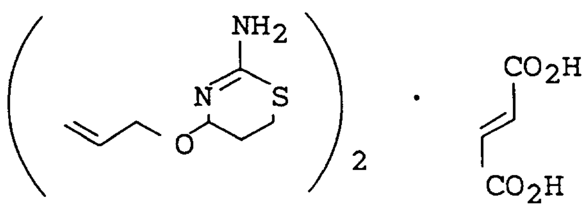

The title compound (3.1 g) was obtained as a white crystal in the same manner as in Example 22 except that allyl alcohol was used instead of benzyl alcohol.

Melting point: 138 - 139°C

$^1$H NMR (300MHz, δppm, DMSO-$d_6$): 1.88 (2H, m), 2.01 (2H, m), 3.03 (2H, m), 3.19 (2H, m), 4.02 (2H, m), 4.19 (2H, m), 4.78 (2H, t, J=3.0Hz), 5.15 (2H, m), 5.30 (2H, m), 5.89 (2H, m), 6.38 (2H, s), 9.25 (2H, broad, s)

FAB (+) MS (low resolution): 173.0

Example 25 (Preparation Method B)

Bis(2-amino-5,6-dihydro-4-(2-phenyl)ethyloxy-4H-1,3-thiazine) fumarate

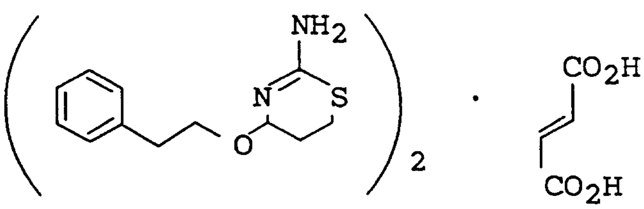

The title compound (3.34 g) was obtained as a white crystal in the same manner as in Example 22 except that phenethyl alcohol was used instead of benzyl alcohol.

Melting point: 138°C

$^1$H NMR (300MHz, δppm, DMSO-$d_6$): 1.87 (4H, m), 2.82 (4H, t, J=7.2Hz), 3.10 (4H, m), 3.64 (2H, m), 3.90 (2H, m), 4.76 (2H, t, J=4.2Hz), 6.45 (2H, s), 7.22 (10H, m)

FAB (+) MS (low resolution): 236

Example 26 (Preparation Method B)

Bis(2-amino-4-ethoxy-5,6-dihydro-6-methyl-4H-1,3-thiazine) fumarate

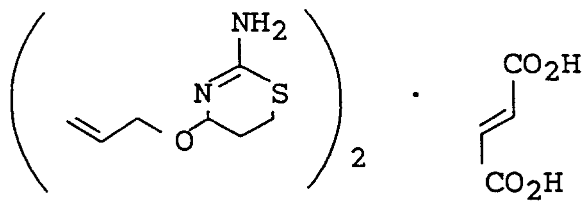

The title compound (0.26 g) was obtained as an amorphous solid in the same manner as in Example 22 except that ethanol instead of benzy lalcohol, crotonaldehyde in stead of acrolein were used and hydroquinone was added.

$^1$H NMR (300 MHz, δppm, DMSO-$d_6$): 1.05 (6H, t, J=6.0 Hz), 1.24 (6H, d, J=6.9 Hz), 1.65 (2H, m), 2.26 (2H, m), 3.53 (2H, m), 3.68 (2H, m), 3.80 (2H, m), 4.92 (2H, m), 6.51 (2H,s)

FAB (+) MS (low resolution): 174

Example 27 (Preparation Method B)

Bis(2-amino-4-benzyloxy-5,6-dihydro-6-methyl-4H-1,3-thiazine), fumarate

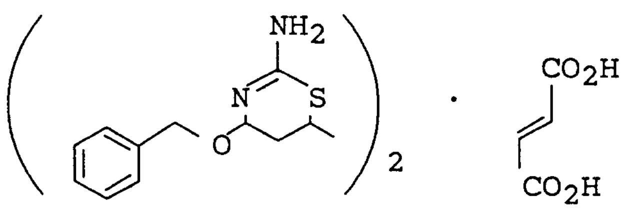

The title compound (0.20 g) was obtained as an amorphous solid in the same manner as in Example 22 except that crotonaldehyde was used instead of acrolein.

$^1$H NMR (300 MHz, δppm, DMSO-$d_6$): 1.34 (6H, t, J=6.6 Hz), 1.69 (2H, m), 2.31 (2H, m), 3.77 (2H, m), 4.58 (2H, d, J=12.0 Hz), 4.70 (2H, d, J=12.0 Hz), 5.00 (2H, m), 6.53 (2H, m), 7.32 (10H, m)

FAB (+) MS (low resolution): 236

Example 28 (Preparation Method C)

2-amino-5,6-dihyro-5-methyl-4H-1,3-thiazine hydrobromide

⟨1⟩: Step 10

2-tert-butylamino-5,6-dihydro-5-methyl-4H-1,3-thiazine

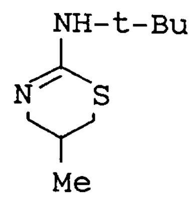

To an ethanol solution (20 ml) of 1-tert-butylthiourea (2.17 g), 1-bromo-3-chloro-2-methylpropane (2.88 ml) and sodium hydrogencarbonate (3.04 g) were added. The resultant mixture was refluxed for 12 hours, concentrated, dissolved in chloroform, and washed successively with an aqueous solution of sodium hydrogencarbonate and an aqueous sodium chloride solution. The chloroform layer was dried over anhydrous sodium sulfate and concentrated, affording the title compound (3.85 g).

⟨2⟩: Step 11

2-amino-5,6-dihydro-5-methyl-4H-1,3-thiazine hydrobromide

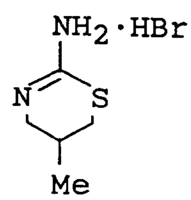

To 2-tert-butylamino-5,6-dihydro-5-methyl-4H-1,3 thiazine (3.85 g) obtained in the aforementioned ⟨1⟩, concentrated hydrobromic acid (20 ml) was added. After heated under reflux for 5.5 hours, the reaction mixture was concentrated, affording the title compound (1.47 g) as a white crystal.

Melting point: 116 - 117°C

$^1H$ NMR (300 MHz, δ ppm, DMSO-$d_6$): 1.04 (3H, d, J=6.0 Hz), 2.00-2.20 (1H, m), 1.65 (2H, m), 2.90-3.20 (2H, m), 3.30-3.50 (2H, m), 8.69 (2H, broad s), 9.62 (1H, s)

FAB (+) MS (low resolution): 130.7

Example 29 (Preparation Method D)

2-amino-5,6-dihydro-6,6-dimethyl-4H-1,3-thiazine hydrochloride

⟨1⟩: Steps 12 and 13

1-(3-methyl-2-butenyl) thiourea

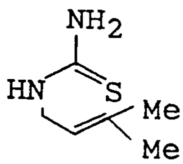

To an acetone solution (82 ml) of ammonium thiocyanate (6.89 g), benzoyl chloride (9.54 ml) was added and heated to 80°C, to give benzoyl isothiocyanate. To the product thus obtained, 2-isopentenylamine hydrochloride (10.0 g) was added and subsequently triethylamine (12.6 ml) was added. After heated under reflux for 2 hours, the reaction mixture was poured into water (300 ml) and formed precipitates were filtrated off, followed by addition of water (123 ml) and sodium hydroxide (12.33 g). The resultant mixture was heated under reflux for 1 hour. After making the reaction mixture into acidic condition by using hydrochloric acid, the reaction mixture was neutralized with a concentrated aqueous ammonia. To the resultant aqueous solution, chloroform was added and the total mass was partitioned into the layers. The chloroform layer was obtained and washed successively with diluted hydrochloric acid, an aqueous sodium hydrogencarbonate solution and water. The chloroform layer was dried over anhydrous sodium sulfate, and then concentrated, affording the title compound (0.92 g).

⟨2⟩: Step 14

2-amino-5,6-dihydro-6,6-dimethyl-4H-1,3-thiazine hydrochloride

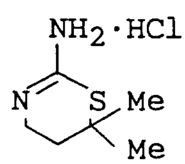

To 1-(3-methyl-2-butenyl) thiourea (0.91 g) obtained in the aforementioned ⟨1⟩, 6N hydrochloric acid (9 ml) was added. The resultant mixture was heated under reflux for 4 hours, and then concentrated, affording the title compound (0.59 g) as a white crystal.

$^1H$ NMR (300 MHz, δppm, $DMSO-d_6$): 1.44 (6H, s) 1.90-2.00 (2H, m), 3.50 (2H, m), 8.95 (2H, broad s), 10.40 (1H, s)

FAB (+) MS (low resolution): 144.9

Example 30 (Preparation Method E)

(S)-2-amino-5,6-dihydro-4-methoxymethyl-4H-1,3-thiazine hydrobromide

⟨1⟩: Step 15

tert-butyl (S)-3-benzyloxycarbonylamino-4-hydroxybutyrate

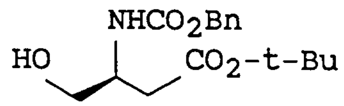

Under a nitrogen atmosphere, N-benzyloxycarbonyl-L-aspartic acid β-tert-butyl ester was dissolved in tetrahydrofuran (50 ml). After the solution was cooled to -18°C, a borane-tetrahydrofuran complex (1M tetrahydrofuran solution: 150 ml) was added thereto. The resultant mixture was stirred for overnight at room temperature. To the reaction mixture, water was added and the resultant solution was extracted with ethyl acetate. The organic layer was washed successively with an aqueous potassium hydrogensulfate solution, an aqueous sodium hydrogencarbonate solution, and a saturated aqueous solution of sodium chloride. The organic layer was dried over magnesium sulfate and concentrated under reduced pressure, affording the title compound (23.0 g).

⟨2⟩: Step 16

tert-butyl (S)-3-benzyloxycarbonylamino-4-methoxybutyrate

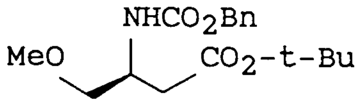

The tert-butyl (S)-3-benzyloxycarbonylamino-4-hydroxybutyrate (23.0 g) obtained in the aforementioned ⟨1⟩ was dissolved in acetonitrile (120 ml), and then, methyl iodide (45 ml) and silver oxide (25 g) were added thereto. The resultant mixture was heated under reflux for a day. The insoluble material was filtered off and the filtrate was concentrated under reduced pressure, affording the title compound (17.0 g).

⟨3⟩: Step 17

(S)-3-benzyloxycarbonylamino-4-methoxybutyric acid

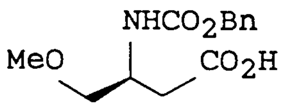

The tert-butyl (S)-3-benzyloxycarbonylamino-4-methoxybutyrate (17.0 g) obtained in the aforementioned ⟨2⟩ was dissolved in a 4N hydrogen chloride-1,4-dioxane solution. The resultant solution was stirred under ice cooling for 2 hours

and further stirred at room temperature for overnight. The reaction mixture was concentrated under reduced pressure, affording the title compound (15.0 g).

⟨4⟩: Step 18

(S)-3-benzyloxycarbonylamino-4-methoxy-1-butanol

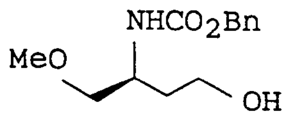

Under a nitrogen atmosphere, (S)-3-benzyloxycarbonylamino-4-methoxybutyric acid (15 g) obtained in the aforementioned ⟨3⟩ was dissolved in tetrahydrofuran (150 ml). After triethylamine (10 ml) was added thereto, the reaction mixture was cooled to 0°C, and then, ethyl chlorocarbonate (1.6 ml) was further added thereto, followed by stirring for 2 hours at room temperature. After the reaction mixture was cooled at 0°C, an aqueous solution (30 ml) of sodium borohydride (7 g) was added thereto. The reaction mixture was stirred for 2 hours at room temperature, and then, a saturated aqueous solution of potassium hydrogensulfate was added to terminate the reaction. After the reaction solution was concentrated, the residue was dissolved in ethyl acetate and washed successively with an aqueous potassium hydrogensulfate solution, an aqueous sodium hydrogencarbonate solution and water. The ethyl acetate layer was dried over anhydrous sodium sulfate and concentrated under reduced pressure. The obtained residue was purified by medium-pressure preparative silica gel column chromatography (eluent; n-hexane : ethyl acetate/1:2), affording the title compound (9.5 g).

⟨5⟩: Step 19

(S)-2-benzyloxycarbonylamino-4-bromo-1-methoxybutane

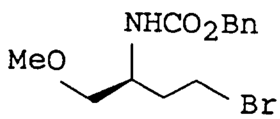

(S)-3-benzyloxycarbonylamino-4-methoxy-1-butanol (9.5 g) obtained in the aforementioned ⟨4⟩ was dissolved in chloroform (20 ml), and then, carbon tetrabromide (16.7 g) and triphenylphosphine (9.7 g) were added thereto. The resultant mixture was stirred for overnight at room temperature. To the reaction mixture, n-hexane was added and insoluble material was filtered off. Thereafter, the filtrate was washed successively with an aqueous sodium hydrogencarbonate solution and a saturated aqueous sodium chloride solution. After the reaction solution was dried over anhydrous magnesium sulfate and concentrated under reduced pressure, the residue thus obtained was purified by medium-pressure preparative silica gel column chromatography (eluent; n-hexane : ethyl acetate/5:1), affording the title compound (6.3 g).

⟨6⟩: Step 20

(S)-2-amino-4-bromo-1-methoxybutane hydrobromide

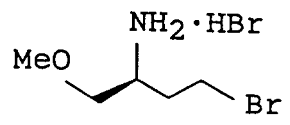

(S)-2-benzyloxycarbonylamino-4-bromo-1-methoxybutane (6.3 g) obtained in the aforementioned ⟨5⟩ was dissolved in 25 % hydrogen bromide in acetic acid solution (50 ml). The resultant solution was stirred for one hour at room temperature and concentrated under reduced pressure. The residue thus obtained was dissolved in ethanol and diluted with isopropyl ether. After the supernatant was removed, the obtained residue was concentrated under reduced pressure, affording the title compound (5.4 g).

⟨7⟩: Step 21

(S)-2-tert-butylamino-5,6-dihydro-4-methoxymethyl-4H-1,3-thiazine

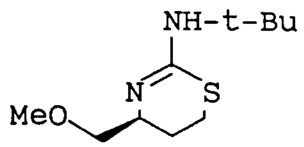

(S)-2-amino-4-bromo-1-methoxybutane hydrobromide (5.4 g) obtained in the aforementioned ⟨6⟩ was dissolved in ethanol (30 ml), followed by addition of tert-butyl isothiocyanate (3.2 ml) and sodium hydrogencarbonate (2.5 g). The resultant mixture was heated under reflux for 6 hours, and then, the reaction mixture was concentrated under reduced pressure. The residue thus obtained was purified by silica gel column chromatography (eluent; chloroform:methanol/96:4), affording the title compound (3.0 g).

⟨8⟩: Step 22

(S)-2-amino-5,6-dihydro-4-methoxymethyl-4H-1,3-thiazine hydrobromide

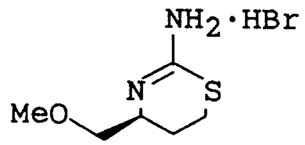

(S)-2-tert-butylamino-5,6-dihydro-4-methoxymethyl-4H-1,3-thiazine (3.0 g) obtained in the aforementioned ⟨7⟩ was dissolved in 25 % hydrogen bromide in acetic acid solution (15 ml). The solution thus obtained was stirred for 1.5 hours at room temperature and then concentrated. The obtained residue was crystallized from ethanol, affording the title compound (360 mg).

Melting point: 157.3 - 158.1°C

$^1H$ NMR (300 MHz, δppm, $D_2O$): 1.85-2.00 (1H, m), 2.20-2.30 (1H, m), 3.10-3.25 (2H, m), 3.37 (3H, s), 3.49 (1H,

dd, J=7.5 and 10.5 Hz), 3.63 (1H, dd, J=4.2 and 10.5 Hz), 3.75 - 3.85 (1H, m)
$[\alpha]_D$: +43.3 (c=1.00, $H_2O$)

Example 31

(R)-2-amino-5,6-dihydro-4-propyl-4H-1,3-thiazine hydrochloride

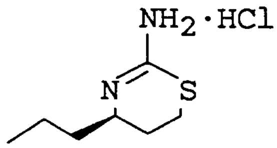

(R)-2-amino-5,6-dihydro-4-propyl-4H-1,3-thiazine hydrobromide (7.63 g) obtained in Example 3 was suspended in chloroform (25 ml), and then, a 10 % aqueous potassium carbonate solution was added thereto. The resultant mixture was vigorously stirred. The organic layer and the water layer was allowed to separate, and then, the water layer was extracted three times with chloroform (50 ml). The combined organic layer was washed with a saturated aqueous sodium chloride solution (50 ml) and concentrated under reduced pressure, affording (R)-2-amino-5,6-dihydro-4-propyl-4H-1,3-thiazine. This product was dissolved in ethanol (50 ml), followed by addition of 4N hydrogen chloride-1,4-dioxane solution (20 ml). The resultant mixture was concentrated under reduced pressure. The residue thus obtained was crystallized from ethanol-diethyl ether, and then, further recrystallized from isopropanol-diisopropyl ether, affording the title compound (3.3 g).

Melting point: 169.7 - 169.9°C

$^1H$ NMR (300 MHz, δppm, DMSO-$d_6$): 0.90 (3H, t, J=6.0 Hz), 1.30-1.60 (4H, m), 1.65-1.80 (1H, m), 2.10-2.25 (1H, m), 3.10-3.30 (2H, m), 3.45-3.60 (1H, m), 8.95 (2H, broad s), 10.29 (1H, broad s)

$[\alpha]_D$: +42.9 (c=0.99, $H_2O$)

It is apparent that the present invention is not restricted to the aforementioned Examples. Accordingly, the compounds listed below, for example, are included in scope of the present invention.

(4aR, 8aS)-2-amino-4a,5,6,7,8,8a-hexahydro-4H-3,1-benzothiazine;
(4aS, 8aS)-2-amino-4a,5,6,7,8,8a-hexahydro-4H-3,1-benzothiazine;
(4aR, 8aR)-2-amino-4a,5,6,7,8,8a-hexahydro-4H-3,1-benzothiazine; and
(4aR, 8aR)-2-amino-4a,5,6,7,8,8a-hexahydro-4H-3,1-benzothiazine.

Hereinbelow, we will describe an NO synthase inhibition activity tested with respect to the thiazine derivative or the thiazepine derivative of the present invention, represented by general formula (I).

Test I:

Activity to inhibit constitutive NO synthase (cNOS) derived from rat cerebellum

The inhibition activity was tested according to a method of P. A. Bush et al. (Biochem, Biophys, Res. Commun., 185, 960-966 (1992)), using [$^3$H]-L-arginine as a substrate and taking the conversion of [$^3$H]-L-arginine to [$^3$H]-L-citrulline as an index of the enzyme activity.

The test was conducted as follows:

First, cNOS was extracted from a rat cerebellum with a 50 mM Tris-HCl buffer solution (pH 7.4) which contains 0.1 mM EDTA (ethylenediamine tetraacetate), 0.1 mM EGTA (ethyleneglycol bis(β-aminoethyl ether)-tetraacetate), 0.5 mM DTT (dithiothreitol), 1 μM PMSF (phenylmethylsulfonyl fluoride), 1 μM bestatin A, and 1 μM leupeptin. To the cNOS thus extracted, a 50 mM Tris-HCl buffer solution (pH 7.4) containing 10 μM L-arginine, 10 μM NADPH, 2 mM calcium chloride and calmoduline (0.1 μg), together with a test compound and [$^3$H]-L-arginine were added. The mixture was incubated for 10 minutes at 25°C. The reaction was terminated by adding 20 mM sodium acetate buffer solution (pH 5.5) containing 1 mM L-citrulline, 2 mM EDTA, and 0.2 mM EGTA. The reaction solution was passed through a cation-exchange resin. This eluate was combined with another eluate which was obtained thereafter by eluting with water. To the combined eluate, a liquid scintillation cocktail was further added and its radioactivity (A) was measured. At the same time, the radioactivity (B) with respect to the case where the test compound was not added and the radioactivity (C) with respect to the case where an enzyme fraction was not added, were respectively measured. Then, the rate of cNOS

inhibition was calculated in accordance with the following equation. The concentration ($IC_{50}$), at which the test compound can inhibit 50 % of the enzyme activity, were also calculated. The results are listed in Table 1.

$$\text{Inhibition rate (\%)} = \{1 - (A - C/B - C)\} \times 100$$

The same test was carried out with respect to known 2-aminothiazoline represented by the following formula, and the result is listed as Comparative Example 1 in Table 1.

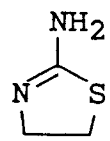

Table 1

| Example | $IC_{50}$(μM) | Example | $IC_{50}$(μM) |
|---|---|---|---|
| Comparative Example 1 | 2 | 16 | 0.2 |
| 1 | 0.02 | 17 | 0.02 |
| 2 | 0.3 | 18 | 0.02 |
| 3 | 0.02 | 19 | 0.04 |
| 4 | 0.03 | 20 | 0.03 |
| 5 | 0.03 | 21 | 0.02 |
| 6 | 0.03 | 22 | 0.1 |
| 7 | 0.04 | 25 | 0.1 |
| 8 | 0.08 | 26 | 0.08 |
| 9 | 0.2 | 27 | 0.4 |
| 10 | 0.03 | 28 | 0.1 |
| 12 | 0.1 | 29 | 0.1 |
| 13 | 0.3 | 30 | 0.03 |
| 15 | 0.6 | 31 | 0.02 |

Test 2:

Activity to inhibit inducible NO synthase (iNOS) derived from rat macrophages

According to partly modified method of Y. Yui et al.(J. Biol. Chem., 266 (19), 12544-12547 (1991)), an iNOS fraction was prepared by applying lipopolysaccharide and interferon-γ to peritoneal macrophages. Thereafter, its enzymatic activity was measured based on the conversion of [$^3$H]-L-arginine to [$^3$H]-L-citrulline, in the same manner as in the case of cNOS.

That is, iNOS was extracted from the macrophages which have been treated with lipopolysaccharide and interferon-γ, with a 50 mM Tris-HCl buffer solution (pH 7.4) containing 0.1 mM EDTA, 0.1 mM EGTA, 0.5 mM DTT, 1 μM PMSF, 1 μM bestatin A, and 1 μM leupeptin. To the iNOS thus extracted, a 50 mM Tris-HCl buffer solution (pH 7.4) containing 150 μM L-arginine, 5 mM NADPH, 5 mM DTT, 0.5 mM 5,6,7,8-tetrahydro-L-biopterin and 5 μM FAD was added together with a test compound and [$^3$H]-L-arginine. The mixture was incubated for 30 minutes at 37°C. Thereafter, the reaction was terminated by adding a 25 mM sodium acetate buffer solution (pH 5.5) containing 1.25 mM L-citrulline, 2.5 mM EDTA and 0.25 mM EGTA. The reaction solution was passed through a cation-exchange resin. The obtained eluate was

combined with the additional eluate obtained thereafter by eluting with water, and the radioactivity of the combined eluate was measured. The inhibition rate of the test compound on iNOS was calculated in the same manner as in Test 1. The concentration ($IC_{50}$) of the test compound required for inhibiting 50 % of the enzyme activity, were calculated. The results are listed in Table 2.

The same test was carried out with respect to known 2-aminothiazoline represented by the following formula, and the result is listed as Comparative Example 1 in Table 2.

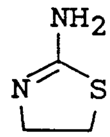

Table 2

| Example | $IC_{50}$(μM) | Example | $IC_{50}$(μM) |
|---|---|---|---|
| Comparative Example 1 | 2 | 18 | 0.01 |
| 1 | 0.3 | 19 | 0.03 |
| 3 | 0.01 | 20 | 0.03 |
| 4 | 0.02 | 21 | 0.03 |
| 5 | 0.04 | 22 | 0.6 |
| 6 | 0.07 | 25 | 0.3 |
| 7 | 0.07 | 26 | 0.09 |
| 8 | 0.07 | 27 | 0.4 |
| 10 | 0.6 | 28 | 0.2 |
| 12 | 0.07 | 29 | 0.2 |
| 16 | 0.5 | 30 | 0.02 |
| 17 | 0.05 | 31 | 0.01 |

Test 3:

Blood pressure elevating activity in rat by inhibition of endothelium nitric oxide synthase (eNOS)

A rat was anesthetized with by peritoneal injection of sodium barbital (300 mg/kg) and catheters were inserted into a femoral artery and a femoral vein.

Blood pressure was measured through the catheter inserted in the femoral artery and a test compound was administered through the catheter inserted in the femoral vein. The dose ($ED_{25mmHg)}$ of the test compound to elevate a mean blood pressure by 25 mmHg was calculated and listed in Table 3.

Table 3

| Example | $ED_{25}$(μg/kg) |
|---|---|
| 3 | 200 |
| 21 | 60 |
| 30 | 150 |
| 31 | 460 |

Test 4:

Effect on rat cerebral ischemia model by inhibition of neuronal NOS (nNOS): reperfusion model

A rat (weight: 300 g) was fasted overnight and a middle cerebral artery of the rat was occluded and then reperfused to prepare a cerebral ischemia model, according to the method of Koizumi et al. (Jpn. J. Stroke, 8, 1-8 (1986)). More specifically, a rat breathing naturally was immobilized under anesthesia with a laughing gas - oxygen mixed gas and halothane, and then, underwent cervical median dissection. A silicon-coated nylon thread was inserted into an internal carotid artery from a branched portion at which a right external carotid artery and the internal carotid artery is divided, thereby blocking the middle cerebral artery for 1.5 hours. Thereafter, the occluding of the middle cerebral artery was reperfused by removing a thread. After 48 hours, 6 coronal sections were prepared and stained by means of a triphenyltetrazolium chloride method. Non-stained portions were determined as infarct area. The test compound was intravenously injected 10 minutes before the occlusion of middle cerebral artery, immediately before, and 3 hours after reperfusion. The inhibitory effect of each compound was determined. The results are listed in Table 4.

Table 4

| Example | Inhibitory Effect (dose; μg/kg) |
|---|---|
| 3 | ○ (10) |
| 21 | ○ (3) |
| 30 | ○ (10) |

Test 5:

Effect on rat cerebral ischemia model by neuronal NOS (nNOS) inhibition: permanent occlusion model

According to a partly modified method of Tamura et al. (J. Cereb. Blood Flow Matab., 1, 53-60 (1981)), an cerebral ischemia model was established by permanently blocking a rat middle cerebral artery. That is, a rat was anesthetized with laughing gas - oxygen and halothane, and then, a hole was formed at a cranial bone in the periphery of oval foramen, thereby exposing the middle cerebral artery. Subsequently, a middle cerebral artery was clipped and coagulated, thereby established a permanent occlusion model. After 24 hours, a brain was excised out and cerebral infarct area were determined by use of triphenyltetrazolium chloride. A test compound was injected intravenously 5 minutes after the occlusion and subsequently infused for 6 hours. The suppression effect was investigated. The results are shown in Table 5.

Table 5

| Example | Suppression Effect (dose) |
|---|---|
| 31 | ○ (10μg/kg+1μg/kg/min) |

Test 6:

Effect on rat-paw carrageenin edema model by inhibition of inducible NO synthase (iNOS)

To a rat's left leg, 1 % carrageenin solution was subcutaneously administered to induce a paw edema. 4 hours after the induction, paw volume was measured. A test compound was orally administered 1 hour prior to the carrageenin administration. The suppression effect of the test compound was determined. The results are shown in Table 6.

Table 6

| Example | Suppression Effect ($ED_{50}$; mg/kg) |
|---|---|
| 3 | ○ (9) |
| 21 | ○ (0.5) |

As is apparent from the aforementioned experiments, a thiazine or thiazepine compound represented by general formula (I) of the present invention has an excellent NO synthase inhibition activity. Hence, these compounds can be used in the treatment for disorders related to excessively produced nitric oxide, such as cardiopathy and encephalopathy ascribed to infarction and ischemia, in particular, encephalopathy caused by ischemia; shock such as endotoxic, hemorrhagic or cardiac shock; and inflammatory disorders such as acute inflammations, chromatic disorders attributable to autoimmune diseases e.g., rheumatism. Particularly, these compounds can be used in the treatment for cerebral ischemia, in other words, various disorders associated with cerebral ischemia and post-ischemia reperfusion injury.

## Claims

1. A 2-amino-5,6-dihydro-4H-1,3-thiazine derivative or a 2-amino-4,5,6,7-tetrahydro-1,3-thiazepine derivative represented by the following formula (I), or a pharmaceutically acceptable salt thereof:

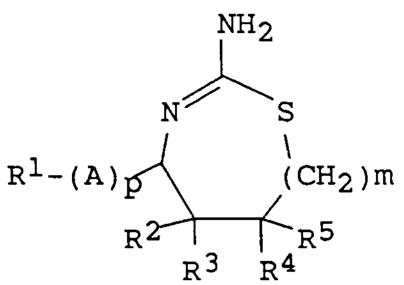

(I)

characterized in that

- $R^1$ is a hydrogen atom, a substituted or unsubstituted alkyl group, a cycloalkyl group, a substituted or unsubstituted alkenyl group, a substituted or unsubstituted alkynyl group, a substituted or unsubstituted aryl group, a substituted or unsubstituted heteroaryl group, or a substituted or unsubstituted aralkyl group;
- $R^2$, $R^3$, $R^4$, and $R^5$, which may be the same or different, respectively is a hydrogen atom, a substituted or unsubstituted alkyl group, or $R^1$ together with $R^2$ or $R^3$ may represent

$$-(CH_2)_n-$$

where n is an integer from 1 to 6;
- A is an oxygen atom, a sulfur atom, or an NH group;
- m is 0 or 1; and

- p is 0 or 1, with the proviso that the following compounds (a) to (f) are excluded from the scope of the Formula (I).

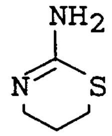
,
(a)

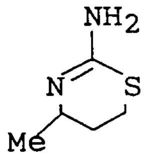
,
(b)

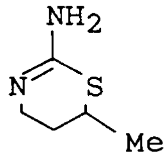
,
(c)

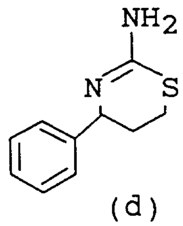
,
(d)

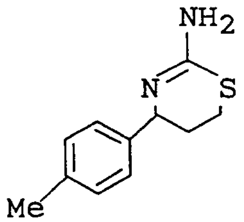
,
(e)

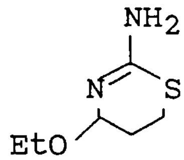
(f)

2. The thiazine derivative, thiazepine derivative, or pharmaceutically acceptable salt thereof according to claim 1, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;
- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;
- 2-amino-4-benzyloxy-5,6-dihydro-4H-1,3-thiazine;
- 2-amino-4-cyclopropylmethoxy-5,6-dihydro-4H-1,3-thiazine;
- 4-allyloxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;
- 2-amino-5,6-dihydro-5-methyl-4H-1,3-thiazine; and
- 2-amino-5,6-dihydro-6,6-dimethyl-4H-1,3-thiazine.

3. The thiazine derivative, thiazepine derivative, or pharmaceutically acceptable salt thereof according to claim 2, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-1-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

4. The thiazine derivative, thiazepine derivative, or pharmaceutically acceptable salt thereof according to claim 3, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

5. The thiazine derivative, thiazepine derivative, or pharmaceutically acceptable salt thereof according to claim 4, characterized in that said thiazine- or thiazepine-derivative is selected from the group consisting of the following compounds:

- (R)-2-amino-5,6-dihydro-4-propyl-4H-1,3-thiazine; and
- (S)-2-amino-5,6-dihydro-4-methoxymethyl-4H-1,3-thiazine.

6. The thiazine derivative or pharmaceutically acceptable salt thereof according to claim 5, characterized in that said thiazine derivative is (R)-2-amino-5,6-dihydro-4- propyl-4H-1,3-thiazine.

7. The thiazine derivative or pharmaceutically acceptable salt thereof according to claim 5, characterized in that said thiazine derivative is (S)-2-amino-5,6-dihydro-4- methoxymethyl-4H-1,3-thiazine.

8. A pharmaceutical composition comprising a sufficient amount a thiazine- or thiazepine-derivative represented by the following formula (I) or a pharmaceutically acceptable salt thereof to inhibit a nitric oxide synthase activity, and a pharmaceutically acceptable carrier:

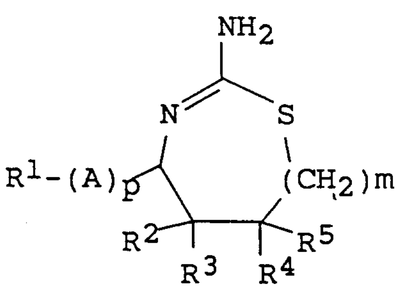

(I)

characterized in that $R^1$, $R^2$, $R^3$, $R^4$, $R^5$, A, m and p respectively have the same meanings as defined above, with the proviso that the case where m and p are simultaneously 0, $R^1$, $R^2$, $R^3$, $R^4$ are simultaneously a hydrogen atom, and $R^5$ is a methyl group, that is, the case representing the following compound (c) is excluded from the scope of the formula (I);

(C) 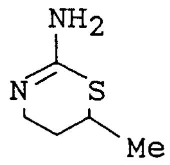

9. The pharmaceutical composition according to claim 8, characterized in that in said general formula (I), p is 0, $R^1$ is a hydrogen atom, a substituted or unsubstituted alkyl group, a substituted or unsubstituted aryl group, a substituted or unsubstituted aralkyl group, or $R^1$ together with $R^2$ forms

$$-(CH_2)_n-$$

where n is the same as defined above.

10. The pharmaceutical composition according to claim 8, characterized in that in said general formula (I), p is 1, each of $R^2$, $R^3$, $R^5$ is a hydrogen atom, $R^4$ is a hydrogen atom or a substituted or unsubstituted alkyl group.

11. The pharmaceutical composition according to claim 8, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;
- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;
- 2-amino-4-ethoxy-5,6-dihydro-4H-1,3-thiazine;
- 2-amino-4-benzyloxy-5,6-dihydro-4H-1,3-thiazine;
- 2-amino-4-cyclopropylmethoxy-5,6-dihydro-4H-1,3-thiazine;
- 4-allyloxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;
- 2-amino-5,6-dihydro-5-methyl-4H-1,3-thiazine; and
- 2-amino-5,6-dihydro-6,6-dimethyl-4H-1,3-thiazine.

12. The pharmaceutical composition according to claim 11, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

**13.** The pharmaceutical composition according to claim 12, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

**14.** The pharmaceutical composition according to claim 13, characterized in that said thiazine- or thiazepine-derivative is selected from the group consisting of the following compounds:

- (R)-2-amino-5,6-dihydro-4-propyl-4H-1,3-thiazine; and
- (S)-2-amino-5,6-dihydro-4-methoxymethyl-4H-1,3-thiazine.

**15.** The pharmaceutical composition according to claim 14, characterized in that said thiazine- or thiazepine-derivative is (R)-2-amino-5,6-dihydro-4-propyl-4H-1,3-thiazine.

**16.** The pharmaceutical composition according to claim 14, characterized in that said thiazine- or thiazepine-derivative is (S)-2-amino-5,6-dihydro-4-methoxymethyl-4H-1,3-thiazine.

**17.** Use of a compound represented by the following formula (I) or a pharmaceutically acceptable salt thereof in preparing a medicament for treating a subject suffering an abnormal symptom ascribed to an excessive level of in-vivo nitric oxide synthesis:

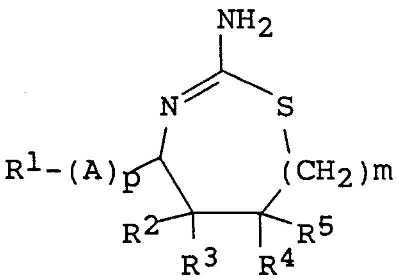

(I)

where

$R^1$, $R^2$, $R^3$, $R^4$, $R^5$, A, m and p respectively have the same meanings as defined above.

**18.** The use according to claim 17, characterized in that in said formula (I), p is 0, $R^1$ is a hydrogen atom, a substituted or unsubstituted alkyl group, a substituted or unsubstituted aryl group, a substituted or unsubstituted aralkyl group, or $R^1$ together with $R^2$ forms

$$-(CH_2)_n-$$

where n is the same as defined above.

**19.** The use according to claim 17, characterized in that in said general formula (1), p is 1, each of $R^2$, $R^3$, $R^5$ is a hydrogen atom, $R^4$ is a hydrogen atom or a substituted or unsubstituted alkyl group.

**20.** The use thereof according to claim 17, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine;
- (S)-2-amino-5,6-dihydro-4-methoxymethyl-4H-1,3-thiazine;
- (R)-2-amino-4-benzyl-5,6-dihydro-4H-1,3-thiazine;
- (S)-2-amino-4-benzyl-5,6-dihydro-4H-1,3-thiazine;
- (S)-2-amino-4-(3-aminopropyl)-5,6-dihydro-4H-1,3-thiazine;
- 2-amino-4,5,6,7-tetrahydro-4-methyl-1,3-thiazepine;
- (R)-2-amino-4,5,6,7-tetrahydro-4-propyl-1,3-thiazepine;
- (S)-2-amino-4,5,6,7-tetrahydro-4-propyl-1,3-thiazepine;
- (R)-2-amino-4-benzyl-4,5,6,7-tetrahydro-1,3-thiazepine;
- 2-amino-5,6-dihydro-5,5-dimethyl-4H-1,3-thiazine;
- 2-amino-5,6-dihydro-4-hydroxy-4H-1,3-thiazine;
- 2-amino-5,6-dihydro-4-methoxy-4H-1,3-thiazine;
- 2-amino-5,6-dihydro-4-propoxy-4H-1,3-thiazine;
- 2-amino-5,6-dihydro-4-isopropoxy-4H-1,3-thiazine;
- 2-amino-4-ethoxy-5,6-dihydro-4H-1,3-thiazine;
- 2-amino-4-benzyloxy-5,6-dihydro-4H-1,3-thiazine;
- 2-amino-4-cyclopropylmethoxy-5,6-dihydro-4H-1,3-thiazine;
- 4-allyloxy-2-amino-5,6-dihydro-4H-1,3-thiazine;
- 2-amino-5,6-dihydro-4-(2-phenyl)ethoxy-4H-1,3-thiazine;
- 2-amino-4-ethoxy-5,6-dihydro-6-methyl-4H-1,3-thiazine;
- 2-amino-4-benzyloxy-5,6-dihydro-6-methyl-4H-1,3-thiazine;
- 2-amino-5,6-dihydro-5-methyl-4H-1,3-thiazine; and
- 2-amino-5,6-dihydro-6,6-dimethyl-4H-1,3-thiazine.

**21.** The use according to claim 20, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-isopropyl-4H-1,3-thiazine;
- (R)-2-amino-5,6-dihydro-4-propyl-4H-1,3-thiazine;
- (S)-2-amino-5,6-dihydro-4-propyl-4H-1,3-thiazine;
- (R)-2-amino-4-butyl-5,6-dihydro-4H-1,3-thiazine;
- (S)-2-amino-4-butyl-5,6-dihydro-4H-1,3-thiazine;
- (R)-2-amino-5,6-dihydro-4-isobutyl-4H-1,3-thiazine;
- (S)-2-amino-5,6-dihydro-4-isobutyl-4H-1,3-thiazine; and
- (S)-2-amino-5,6-dihydro-4-methoxymethyl-4H-1,3-thiazine.

**22.** The use according to claim 21, characterized in that said thiazine- or thiazepine-derivative is selected from the group consisting of the following compounds:

- (S)-2-amino-5,6-dihydro-4-isopropyl-4H-1,3-thiazine;
- (R)-2-amino-5,6-dihydro-4-propyl-4H-1,3-thiazine;
- (R)-2-amino-4-butyl-5,6-dihydro-4H-1,3-thiazine;
- (R)-2-amino-5,6-dihydro-4-isobutyl-4H-1,3-thiazine; and
- (S)-2-amino-5,6-dihydro-4-methoxymethyl-4H-1,3-thiazine.

**23.** The use according to claim 22, characterized in that said thiazine- or thiazepine-derivative is selected from the group consisting of the following compounds:

- (R)-2-amino-5,6-dihydro-4-propyl-4H-1,3-thiazine; and
- (S)-2-amino-5,6-dihydro-4-methoxymethyl-4H-1,3-thiazine.

**24.** The use according to claim 23, characterized in that said thiazine- or thiazepine-derivative is (R)-2-amino-5,6-dihydro-4-propyl-4H-1,3-thiazine.

**25.** The use according to claim 23, characterized in that said thiazine- or thiazepine-derivative is (S)-2-amino-5,6-dihydro-4- methoxymethyl-4H-1,3-thiazine.

**26.** The use according to claim 17, characterized in that said abnormal symptom ascribed to an excessive level of in-vivo nitric oxide synthesis is inflammation.

**27.** The use according to any of claims 18 to 25, characterized in that said abnormal symptom ascribed to an excessive level of in-vivo nitric oxide synthesis is inflammation.

**28.** The use according to claim 17, characterized in that said abnormal symptom ascribed to an excessive level of in-vivo nitric oxide synthesis is a shock symptom.

**29.** The use according to any of claims 18 to 25, characterized in that said abnormal symptom ascribed to an excessive level of in-vivo nitric oxide synthesis is a shock symptom.

**30.** The use according to claim 17, characterized in that said abnormal symptom ascribed to an excessive level of in-vivo nitric oxide synthesis is ischemic encephalopathy.

**31.** The use according to any of claims 18 to 25, characterized in that the abnormal symptom ascribed to an excessive level of in-vivo nitric oxide synthesis is ischemic encephalopathy.

European Patent Office

# EUROPEAN SEARCH REPORT

Application Number
EP 95 11 2468

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | WO-A-95 11231 (G.D.SEARLE) 27 April 1995<br>* claim 1 * | 1-31 | C07D279/06<br>A61K31/54<br>C07D281/02 |
| X | ARCH PHARM BER DTSCH PHARM GES,<br>vol. 301, no. 10, 1968<br>pages 750-762,<br>SCHUBERT ET AL 'Neue Synthesen von 2-Amino-5,6-dihydro-4H-1,3-thiazinen'<br>* page 752; examples 6B-6K * | 1 | |
| X | DE-A-11 76 148 (BAYER) 20 August 1964<br>* column 3, line 47 - column 4, line 33 * | 1 | |
| D,A | WO-A-94 12165 (WELLCOME FOUNDATION) 9 June 1994<br>* claim 1 * | 1-31 | |
| D,A | US-A-3 169 090 (SKALETZKY) 9 February 1965<br>* column 4, line 5; claim 1 * | 1 | |

TECHNICAL FIELDS SEARCHED (Int.Cl.6)

C07D
A61K

The present search report has been drawn up for all claims

| Place of search | Date of completion of the search | Examiner |
|---|---|---|
| MUNICH | 5 March 1996 | Gettins, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)